(19) Europäisches Patentamt
European Patent Office
Office européen des brevets

(11) EP 3 448 357 B1

(12) **EUROPÄISCHE PATENTSCHRIFT**

(45) Veröffentlichungstag und Bekanntmachung des Hinweises auf die Patenterteilung:
**26.02.2020 Patentblatt 2020/09**

(21) Anmeldenummer: **17720426.0**

(22) Anmeldetag: **24.04.2017**

(51) Int Cl.:
***A61Q 5/12*** *(2006.01)* ***A61K 8/86*** *(2006.01)*
***A61K 8/898*** *(2006.01)* ***A61K 8/06*** *(2006.01)*

(86) Internationale Anmeldenummer:
**PCT/EP2017/059644**

(87) Internationale Veröffentlichungsnummer:
**WO 2017/186633 (02.11.2017 Gazette 2017/44)**

(54) **WÄSSRIGE EMULSIONEN AUS CARBAMATO-FUNKTIONALISIERTEN ORGANOPOLYSILOXANEN**

AQUEOUS EMULSION BASED ON CARBAMATO-MODIFIED ORGANOPOLYSILOXANES

EMULSION AQUEUSE COMPRENANT DES ORGANOPOLYSILOXANES MODIFIÉES PAR DES GROUPES CARBAMATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.04.2016 DE 102016207060**

(43) Veröffentlichungstag der Anmeldung:
**06.03.2019 Patentblatt 2019/10**

(73) Patentinhaber: **Wacker Chemie AG**
**81737 München (DE)**

(72) Erfinder:
- **BREHM, Christof**
**84489 Burghausen (DE)**
- **BEER, Gerhard**
**84489 Burghausen (DE)**
- **LIMMER, Werner**
**84568 Pleiskirchen (DE)**

(74) Vertreter: **Deffner-Lehner, Maria et al**
**Wacker Chemie AG**
**Zentralbereich Patente, Marken und Lizenzen**
**Hanns-Seidel-Platz 4**
**81737 München (DE)**

(56) Entgegenhaltungen:
**EP-A2- 1 972 330**
**US-A- 4 620 878**
**US-A- 5 389 364**
**US-A1- 2011 097 567**
**US-A1- 2012 270 985**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 448 357 B1

Printed by Jouve, 75001 PARIS (FR)

## Beschreibung

Die Erfindung betrifft wässrige Emulsionen aus Carbamato-funktionalisierten Organopolysiloxanen, deren Herstellung und deren Verwendung in kosmetischen Zusammensetzungen.

Silicone als Bestandteile von Kosmetik-Zusammensetzungen sind seit langem bekannt. Das gleiche gilt für Carbamato-funktionalisierte Polydiorganosiloxane, wie sie beispielsweise in EP 639 369 A1 beschrieben sind. Die dort verwendeten Carbamato-funktionalisierten Polydiorganosiloxane (hergestellt durch Umsetzung von Amino-funktionalisierten Polydiorganosiloxanen mit Ethylen- und/oder Propylencarbonat) zur Konditionierung von Haaren werden in Substanz der haarkonditionierungsmittelhaltigen Zusammensetzung hinzugefügt. In klaren/transparenten Kosmetik-Produkten ist der Einsatz nichtformulierter Polydiorganosiloxane häufig ausgeschlossen, da sich die Kosmetik-Zusammensetzungen dadurch häufig eintrüben und auch an Stabilität einbüßen. Dies ist insbesondere bei Polysiloxanen höherer Aminzahl oder bei Polydiorganosiloxanen, die aus Amino-funktionalisierten Polydiorganosiloxanen höherer Aminzahl hergestellt wurden, zu beobachten. In den Beispielen der EP 639 369 A1 ist dies der Fall (Aminzahl > 0,5 mmol/g). In EP 1 972 330 wird ebenso die Verwendung von Carbamato-funktionalisierten Polydiorganosiloxanen in Substanz in kosmetischen Produkten beschrieben, wobei die Carbamato-funktionalisierten Polydiorganosiloxane durch die Umsetzung mit Glycerincarbonat hergestellt werden.

Aus WO 2009/150213 sind analoge Produkte (hergestellt durch Umsetzung von Amino-funktionalisierten Polydiorganosiloxanen höherer Aminzahl von größer 0,5 mmol/g und Glycerincarbonat) für das Finishing organischer Fasern und Textilien bekannt. Eine Möglichkeit Organopolysiloxane in kosmetische Zusammensetzungen einzuarbeiten, stellt deren Darreichungsform in Form von Emulsionen, insbesondere in Form von Mikroemulsionen für klare/transparente kosmetische Zusammensetzungen, dar. Mikroemulsionen sind thermodynamisch stabile Mischungen aus Wasser (wässrige Phase), Öl (nicht mit Wasser mischbare Phase) und Tensid (Solubilisator).

Mikroemulsionen, bei denen die Öl-Phasen maßgeblich durch Polysiloxane gebildet werden, sind bekannt.

Aus US 4,620,878 ist die Herstellung von Emulsionen und Mikroemulsionen, die Amino-funktionelle Polydiorganosiloxane enthalten, bekannt. Nach der dort beschriebenen Methode wird zuerst ein Konzentrat bestehend aus Tensid, Polydiorganosiloxan und geringen Mengen Wasser hergestellt, das mit Wasser unter Bildung der Mikroemulsion verdünnt wird.

In US 6,153,569 wird die Verwendung von Mikroemulsionen mit Amino-funktionalisierten Polydiorganosiloxanen, um klare Shampoo-Zusammensetzungen zu erhalten, beschrieben. Es werden Amino-funktionalisierte Polydiorganosiloxane höherer Aminzahl (> 0,16 mmol/g) verwendet, da die Herstellung von Mikroemulsionen mit Amino-funktionalisierten Polydiorganosiloxanen geringerer Aminzahl nur schwierig zu realisieren ist.

Dokument US2012/0270985 offenbart wässrige Emulsionen enthaltend funktionalisierte Organopolysiloxane, nicht-ionische, Polyethylenoxid-haltige Emulgatoren, Wasser, wobei die Emulsionen Partikelgrößen von kleiner oder gleich 100 nm (D50) aufweisen. Die Emulsionen von D1 sind stabil und klar.

Wenn die Polydiorganosiloxane durch hydrophile Gruppen, insbesondere mit einer größeren Zahl an Hydroxy-Gruppen, funktionalisiert sind, kann die Darstellung von Mikroemulsionen gelingen. Ein Beispiel ist z.B. die Mikroemulsion des Reaktionsprodukts aus Amino-funktionalisiertem Polydiorganosiloxan der Aminzahl von 0,98 mmol/g und Glycerincarbonat, wie sie in WO 2009/150213 A1 beschrieben ist. Entscheidend hier ist das Maß an Hydroxy-Gruppen bezogen auf das Silicon. In dem in WO 2009/150213 A1 genannten Beispiel sind es ca. 3,13 Gew.-% HO-Gruppen.

Es bestand die Aufgabe, wässrige Emulsionen von Carbamato-funktionalisierten Organopolysiloxanen mit kleiner Partikelgröße bereitzustellen, wobei die Organopolysiloxane nur einen niedrigen Gehalt an Hydroxygruppen aufweisen, die Emulsionen klar sind und geeignet sind zur Herstellung von klaren kosmetischen Zusammensetzungen, die auch nach Lagerung keine bzw. nur sehr geringe Viskositätsänderungen aufweisen. Die Aufgabe wurde durch die Erfindung gelöst.

Gegenstand der Erfindung sind wässrige Emulsionen, wie in Anspruch 1 offenbart, enthaltend

(A) Carbamato-funktionalisierte Organopolysiloxane, die durchschnittlich je Molekül mindestens eine Carbamatofunktionelle Gruppe Y der Formel

$$-R^4-[NX-R^5-]_nNX-H$$

enthalten, wobei
X gleich oder verschieden ist und ein Wasserstoffatom oder einen Rest Z der Formel

$$-CO-O-CHR^6-CH_2-OH$$

oder

$-CO-O-CH_2-CHR^6-OH$

bedeutet, wobei durchschnittlich je Molekül mindestens ein Rest X ein Rest Z ist,
wobei $R^4$ gleich oder verschieden ist und einen zweiwertigen, Si-C-gebundenen Kohlenwasserstoffrest mit 1 bis 18 C-Atomen bedeutet,
$R^5$ gleich oder unterschiedlich ist und einen zweiwertigen Kohlenwasserstoffrest mit 1 bis 6 C-Atomen bedeutet,
$R^6$ gleich oder unterschiedlich ist und ein Wasserstoffatom oder einen einwertigen Kohlenwasserstoffrest mit 1 bis 36 C-Atomen bedeutet, und
n gleich 1, 2, 3 oder 4, vorzugsweise 1, ist,

(B) nicht-ionische, Polyethylenoxid-haltige Emulgatoren, die mehr als 40 Ethylenoxid-Einheiten der Formel $-CH_2-CH_2-O-$enthalten, und einen HLB-Wert von größer oder gleich 15 aufweisen,
und
(C) Wasser,
wobei die Emulsionen Partikelgrößen von kleiner oder gleich 100 nm (D50) aufweisen.

Die erfindungsgemäßen Emulsionen enthalten vorzugsweise mindestens 5 Gew.-%, bevorzugt mindestens 10 Gew.-%, und vorzugsweise höchstens 50,0 Gew.-%, bevorzugt höchstens 35 Gew.-%, besonders bevorzugt höchstens 25 Gew.-%, Carbamato-funktionalisierte Organopolysiloxane (A).

Die erfindungsgemäßen Emulsionen enthalten vorzugsweise mindestens 0,5 Gew.-%, bevorzugt mindestens 0,8 Gew.-%, besonders bevorzugt mindestens 1,2 Gew.-%, und vorzugsweise höchstens 20 Gew.-%, bevorzugt höchstens 15 Gew.-%, besonders bevorzugt höchstens 10 Gew.-%, nicht-ionische, Polyethylenoxid-haltige Emulgatoren (B).

Die erfindungsgemäßen Emulsionen enthalten vorzugsweise mindestens 1 Gew.-%, bevorzugt mindestens 5 Gew.-%, insbesondere mindestens 10 Gew.-%, und vorzugsweise höchstens 94,5 Gew.-%, bevorzugt höchstens 85 Gew.-%, insbesondere höchstens 80 Gew.-% Wasser (C).

Die erfindungsgemäßen Emulsionen weisen vorzugsweise Partikelgrößen (D50) von kleiner oder gleich 80 nm, bevorzugt kleiner oder gleich 50 nm, besonders bevorzugt kleiner oder gleich 40 nm, auf.

Die erfindungsgemäßen wässrigen Emulsionen können gegebenenfalls neben den

(A) Carbamato-funktionalisierten Organopolysiloxanen,
(B) nicht-ionischen, Polyethylenoxid-haltigen Emulgatoren, die mehr als 40 Polyethylenoxid-Einheiten der Formel $-CH_2-CH_2-O-$ und einen HLB-Wert von größer oder gleich 15 aufweisen, und
(C) Wasser
weitere Inhaltsstoffe enthalten, wie
(D) weitere ionische oder nicht-ionische Emulgatoren oder deren Mischungen mit einem HLB-Wert von jeweils kleiner als 15,
(E) nichtwässrige Lösemittel oder Co-Emulgatoren und
(F) Hilfsstoffe, wie pH-Regulierungsmittel, Salze, Schauminhibitoren, Verdicker und/oder Schutzkolloide, Konservierungsmittel, Desinfektionsmittel, Netzmittel, Korrosionsinhibitoren, Farbstoffe, Duftstoffe oder deren Mischungen.

Carbamato-funktionalisierte Polydiorganosiloxane (A) sind seit langem bekannt und beispielsweise in JP 2047371 A2 beschrieben.

In den erfindungsgemäßen Zusammensetzungen werden vorzugsweise Carbamato-funktionalisierte Organopolysiloxane (A) eingesetzt, die folgende Struktureinheiten enthalten:

$\underline{M}$ $[R^1_2R^2SiO_{1/2}]$

und/oder

$\underline{M'}$ $[R^1_2(Y)SiO_{1/2}]$

und

$\underline{D}$ $[R^1_2SiO_{2/2}]$

und/oder

D' $[R^2(Y)SiO_{2/2}]$

und/oder

T' $[(Y)SiO_{3/2}]$

und gegebenenfalls

T $[R^1SiO_{3/2}]$

und/oder

Q $[SiO_{4/2}]$

mit der Maßgabe, dass durchschnittlich je Molekül mindestens eine Struktureinheit mit einer Carbamato-funktionellen Gruppe Y enthalten ist und wobei durchschnittlich je Molekül mindestens eine Gruppe Y einen Rest Z enthält, wobei

$R^1$ gleich oder verschieden ist und einen einwertigen Si-C-gebundenen Kohlenwasserstoffrest mit 1 bis 18 C-Atomen bedeutet,
$R^2$ gleich oder unterschiedlich ist und gleich Rest $R^1$ oder eine Hydroxygruppe -OH oder eine Alkoxy-Gruppe der Formel $-O-R^3$ bedeutet, wobei $R^3$ ein gegebenenfalls substituierter Alkylrest mit 1-8 C-Atomen ist, und
Y die oben dafür angegebene Bedeutung hat.

In den erfindungsgemäßen Zusammensetzungen werden bevorzugt Carbamato-funktionalisierte Polydiorganosiloxane (A) der folgenden Formel eingesetzt

$$[R^1{}_2R^2SiO_{1/2}]_2[R^2(Y)SiO_{2/2}]_k[R^1{}_2SiO_{2/2}]_m \qquad (I),$$

wobei

$R^1$, $R^2$ und Y die oben dafür angegebene Bedeutung haben, wobei durchschnittlich je Molekül mindestens eine Gruppe Y einen Rest Z enthält,
m eine ganze Zahl und mindestens 40, vorzugsweise mindestens 65, bevorzugt mindestens 110, und höchstens 1000, vorzugsweise höchstens 800, bevorzugt höchstens 500, ist
k eine ganze Zahl und mindestens 1, vorzugsweise mindestens 2, und höchstens 15, vorzugsweise höchstens 10, bevorzugt höchstens 7, ist,
wobei das Verhältnis von m zu k mindestens 65, vorzugsweise mindestens 100, bevorzugt mindestens 130, und höchstens 1000, vorzugsweise höchstens 800, bevorzugt höchstens 600 ist.

Gegebenenfalls können in den Carbamato-funktionalisierten Polydiorganosiloxanen (A) der Formel (I) auch geringe Mengen an Struktureinheiten T oder Q enthalten sein.
Bei den Carbamato-funktionalisierten Organopolysiloxanen (A), insbesondere Carbamato-funktionalisierten Polydiorganosiloxanen (A), sind vorzugsweise mindestens 5 Mol%, bevorzugt mindestens 15 Mol%, besonders bevorzugt mindestens 20 Mol%, und weniger als 50 Mol% der N-gebundenen Reste X in den Gruppen Y nicht ein Wasserstoffatom, sondern haben die Bedeutung des Restes Z.
Dies bedeutet, dass mindestens 5 Mol% und weniger als 50 Mol%, der Aminogruppen mit Resten Z, also Carbamatogruppen, funktionalisiert sind.
Es kann eine Art von erfindungsgemäßen Carbamato-funktionalisierten Organopolysiloxane (A) eingesetzt werden oder Mischungen von zwei oder mehreren Arten.
Die in den erfindungsgemäßen Zusammensetzungen eingesetzten Carbamato-funktionalisierten Organopolysiloxane (A) werden vorzugsweise durch Umsetzung von Amino-funktionalisierten Organopolysiloxanen (A'), die durchschnittlich je Molekül mindestens eine Gruppe Y' der Formel

$$-R^4-[NH-R^5-]_nNH_2$$

enthalten, wobei $R^4$, $R^5$ und n die oben dafür angegebene Bedeutung haben,

mit cyclischen Carbonaten hergestellt.

Zur Herstellung der Carbamato-funktionalisierten Organopolysiloxane (A) werden bevorzugt Amino-funktionalisierte Polydiorganosiloxane (A') der folgenden Formel eingesetzt

$$[R^1_2R^2SiO_{1/2}]_2[R^2(Y')SiO_{2/2}]_k[R^1_2SiO_{2/2}]_m \quad (I'),$$

wobei

$R^1$, $R^2$ , m, und k die oben angegebene Bedeutung aufweisen,
Y' eine Gruppe der allgemeinen Formel

$$-R^4-[NH-R^5-]_nNH_2$$

ist, wobei $R^4$, $R^5$ und n die oben dafür angegebene Bedeutung haben.

Als cyclische Carbonate werden vorzugsweise solche der folgenden Formel eingesetzt

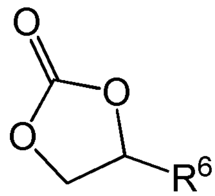

wobei,
$R^6$ die oben dafür angegebene Bedeutung hat.

Zur Herstellung der Carbamato-funktionalisierten Organopolysiloxane (A) wird die Menge an eingesetztem cyclischem Carbonat so ausgewählt, dass im hergestellten Carbamato-funktionalisierten Organopolysiloxan (A) vorzugsweise mindestens 5 mol%, bevorzugt mindestens 15 mol%, besonders bevorzugt mindestens 20 mol%, und vorzugsweise weniger als 100 mol%, bevorzugt weniger als 75 mol%, besonders bevorzugt weniger als 50 mol% der N-gebundenen Reste X in den Gruppen Y zu Resten Z umgesetzt werden.

Durch die Umsetzung von Amino-funktionalisierten Organopolysiloxanen (A') mit aus cyclischen Carbonaten erhaltenen Carbamato-funktionalisierten Organopolysiloxanen (A) werden Hydroxy-Gruppen generiert. Die Menge an Hydroxygruppen (Gew.-% OH) in den Carbamato-funktionalisierten Organopolysiloxanen, die durch diese Funktionalisierung eingebracht wird, ist vorzugsweise mindestens 0,002 Gew-%, bevorzugt mindestens 0,02 Gew.-%, besonders bevorzugt mindestens 0,03 Gew.-%, und vorzugsweise höchstens 0,7 Gew.-%, bevorzugt höchstens 0,32 Gew.-%, besonders bevorzugt höchstens 0,17 Gew.-%.

Beispiele für Kohlenwasserstoffreste $R^1$ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste.

Bevorzugte Beispiele für $R^1$ sind der Methyl-, Ethyl-, Octyl- und Dodecylrest. Ein besonders bevorzugtes Beispiel für $R^1$ ist der Methylrest.

Beispiele für Kohlenwasserstoffreste $R^2$ sind die Reste wie für Rest $R^1$ beschrieben oder eine Hydroxygruppe -OH oder eine Alkoxy-Gruppe der Formel $-O-R^3$ mit $R^3$ gleich einem Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest. Bevorzugte Beispiele für $R^2$ sind der Methylrest, der Ethylrest, die Hydroxygruppe, der Methoxyrest und der Ethoxyrest.

Beispiele für $R^4$ sind zweiwertige Kohlenwasserstoffreste wie die Methylengruppe, die 1,2-Ethylengruppe, die 1,3-Propylengruppe, die 1,3-Butylengruppe, die 1,4-Butylengruppe, die 1,5-Pentylengruppe, die 1,6-Hexylengruppe. Besonders bevorzugte Beispiele sind die 1,3-Propylengruppe und die 1,3-Butylengruppe.

Beispiele für $R^5$ sind zweiwertige Kohlenwasserstoffreste wie die 1,2-Ethylengruppe, die 1,3-Propylengruppe, die 1,3-Butylengruppe, die 1,4-Butylengruppe, die 1,5-Pentylengruppe, die 1,6-Hexylengruppe.
Ein besonders bevorzugtes Beispiel ist die 1,2-Ethylengruppe.

$R^6$ ist vorzugsweise ein Wasserstoffatom oder ein einwertiger, gegebenenfalls durch -O- substituierter, Kohlenwasserstoffrest mit 1 bis 10 C-Atomen, bevorzugt mit 1 bis 6 C-Atomen, besonders bevorzugt ein Alkyl- oder Alkoxyalkylrest mit 1 bis 10 C-Atomen, insbesondere mit 1 bis 6 C-Atomen.

Bevorzugte Beispiele für die Reste Y' sind

- $(CH_2)_2NH(CH_2)_2NH_2$,
- $(CH_2)_3NH(CH_2)_2NH_2$,
- $(CH_2)_3NH(CH_2)_3NH_2$,
- $(CH_2\text{-}CH(CH_3)\text{-}CH_2\text{-})NH(CH)_2NH_2$ und
- $(CH_2)_4NH(CH_2)_4NH_2$.

Besonders bevorzugte Beispiele für die Reste Y' sind

- $(CH_2)_3NH(CH_2)_2NH_2$, und
- $(CH_2\text{-}CH(CH_3)\text{-}CH_2\text{-})NH(CH)_2NH_2$.

Die Indices k und m in Formel (I') werden so gewählt, dass die Viskosität der eingesetzten Amino-funktionalisierten Polydiorganosiloxane (A') bei vorzugsweise mindestens 50 mPas, bevorzugt mindestens 100 mPas, besonders bevorzugt mindestens 250 mPas, jeweils gemessen bei 25°C und einem Schergefälle von 10/s, und vorzugsweise höchstens 100000 mPas, bevorzugt höchstens 50000 mPas, besonders bevorzugt höchstens 10000 mPas, jeweils gemessen bei 25°C und einem Schergefälle von 5/s, liegt.

Das Verhältnis von k und m wird so gewählt, dass die eingesetzten Amino-funktionalisierten Polydiorganosiloxane (A') eine Aminzahl von vorzugsweise mindestens 0,025 mmol/g, bevorzugt mindestens 0,075 mmol/g, besonders bevorzugt mindestens 0,1 mmol/g, und vorzugsweise höchstens 0,4 mmol/g, bevorzugt höchstens 0,25 mmol/g, besonders bevorzugt höchstens 0,2 mmol/g, aufweisen.

Durch die Umsetzung der Amino-funktionalisierten Organopolysiloxane (A') mit cyclischen Carbonaten reduziert sich die Aminzahl. Die Carbamato-funktionalisierten Organopolysiloxanen (A), insbesondere Carbamato-funktionalisierten Polydiorganosiloxanen (A), weisen somit eine Aminzahl von vorzugsweise mindestens 0 mmol/g, bevorzugt mindestens 0,02 mmol/g, besonders bevorzugt mindestens 0,04 mmol/g, und vorzugsweise höchstens 0,30 mmol/g, bevorzugt höchstens 0,20 mmol/g, besonders bevorzugt höchstens 0,10 mmol/g auf.

Die zur Herstellung der Carbamato-funktionalisierten Polydiorganosiloxane (A) eingesetzten cyclischen Carbonate sind entweder kommerziell erhältlich oder können synthetisiert werden, wie beispielsweise in US 3642858 A beschrieben.

Typischerweise werden als cyclische Carbonate 1,2-Alkylencarbonate oder Alkoxyalkyl-substituierte Ethylencarbonate eingesetzt.
Bevorzugte cyclische Carbonate sind solche Carbonate mit
$R^6$ gleich einem Wasserstoffatom oder einem einwertigen, gegebenenfalls durch -O- substituierten, Kohlenwasserstoffrest mit 1 bis 10 C-Atomen, besonders bevorzugt mit 1 bis 6 C-Atomen.
Beispiele sind Ethylencarbonat, 1,2-Propylencarbonat, 1,2-Butylencarbonat, 1,2-Pentylencarbonat, 1,2-Hexylencarbonat, 1,2-Octylencarbonat, 1,2-Dodecylencarbonat, 3-Methyl-1,2-butylencarbonat, 3-Methyl-1,2-Pentylencarbonat, 3-Ethyl-1,2-Pentylencarbonat, 4-Methyl-1,2-Pentylencarbonat, 5-Methyl-1,2-Hexylencarbonat, 3-Methoxy-l,2-Propylencarbonat, 3-Ethoxy-1,2-Propylencarbonat, 3-n-Propoxy-l,2-propylencarbonat, 4-Methoxy-1,2-butylencarbonat, 4-Ethoxy-1,2-butylencarbonat, S-Methoxy-3-butylencarbonat, 5-Methoxy-1,2-Heptylencarbonat.
Bevorzugte cyclische Carbonate sind Ethylencarbonat, 1,2-Propylencarbonat, 3-Methoxy-1,2-Propylencarbonat.
Ein besonders bevorzugtes cyclisches Carbonat ist das 1,2-Propylencarbonat.

Bei den in den erfindungsgemäßen wässrigen Emulsionen verwendeten nicht-ionischen, Polyethylenoxid-haltigen Emulgatoren (B) handelt es sich um solche mit einem HLB-Wert von vorzugsweise größer oder gleich 16, bevorzugt größer oder gleich 17 und gleichzeitig einem Gehalt an Ethylenoxid-Einheiten von vorzugsweise größer oder gleich 45, bevorzugt größer oder gleich 50, sowie vorzugsweise kleiner oder gleich 600, bevorzugt kleiner oder gleich 400, besonders bevorzugt kleiner oder gleich 250.

Der HLB-Wert (HLB steht für engl. hydrophilic-lipophilic balance) beschreibt den hydrophilen und lipophilen Anteil von hauptsächlich nichtionischen Tensiden. Der HLB-Wert kann für nichtionische Tenside nach Hans-Dieter Dörfler, Grenzflächen- und Kolloidchemie, VCH, Weinheim, 1994, S. 198, folgendermaßen berechnet werden:

$$\mathrm{HLB} = 20 \times \left(1 - \frac{\mathrm{M_l}}{\mathrm{M}}\right)$$

Dabei ist $M_l$ die Molmasse des lipophilen Anteils eines Moleküls und M die Molmasse des gesamten Moleküls. Der Faktor 20 ist ein frei ausgewählter Skalierungsfaktor.

Die erfindungsgemäßen nichtionischen, Polyethylenoxid-haltigen Emulgatoren (B) sind:

- Alkylpolyglycolether, vorzugsweise solche Alkylresten von 8 bis 20 C-Atomen, wie z.B. Steareth-100 (9005-00-9), Talloweth-50, Talloweth-80 (61791-28-4), Trideceth-50 (24938-91-8),
- Carbonsäurepolyglycolester, insbesondere Fettsäurepolyglycolester, vorzugsweise solche Carbonsäureresten von 8 bis 20 C-Atomen, wie z.B. PEG-75 Oleat, PEG-200 Oleat, PEG-300 Monooleat, PEG-400 Oleat, PEG-150 Laurat, PEG-400 Laurat, PEG-75 Stearat, PEG-100 Stearat, PEG-600 Stearat, PEG-150 Distearat,
- Ethoxylierte Sorbitanfettsäureester, wie z.B. PEG-40 Sorbitanoleat, PEG-80 Sorbitanlaurat,
- Ethoxyliertes Rizinusöl oder hydrierte Varianten, wie z.B. (Bezeichnung nach INCI-Nomenklatur) PEG 75 Castor Oil oder PEG200 Castor Oil bzw. PEG-80 hydrogenated Castor Oil, PEG-100 hydrogenated Castor Oil, PEG-200 hydrogenated Castor Oil,
- Ethoxylierte Fettamine, wie z.B. PEG-100 Talg-alkylamin (61791-44-4), PEG-40 Stearylamin,
- Ethoxylierte Glyceryl Fettsäurecarboxylate, wie z.B. PEG-40 Glyceryllaurat, PEG-200 Glycerylstearat, PEG-200 Glyceryltallowat, PEG-200 hydriertes Glycerylpalmat
- Blockcopolymere aus Ethylenoxid- und Propylenoxid-Einheiten (Polyalkylenblockpolymere wie die sogenannten Poloxamere), wie z.B. PEG-PPG-PEG-Blockpolymer Pluronic® F-108 (HLB >24; $M_n \approx 14600$) (erhältlich bei Sigma-Aldrich),
- Copolymere aus Ethylenoxid- und Propylenoxid-Einheiten, die über einen Ethylendiamin-Kern verbrückt sind (sogenannte Poloxamine), wie z.B. Tetronic 1107 (HLB-Wert: 24; $M_n \approx 15000$) (erhältlich bei Sigma-Aldrich).

Die erfindungsgemäßen nicht-ionischen, Polyethylenoxid-haltigen Emulgatoren (B) können aus einem der o.g. Emulgatoren oder aus einem Gemisch zweier oder mehrerer o.g. Emulgatoren bestehen, wobei sie in reiner Form oder als Lösungen eines oder mehrerer Emulgatoren in Wasser oder organischen Lösungsmitteln eingesetzt werden können.

Als weitere Inhaltsstoffe in den erfindungsgemäßen Emulsionen können (D)
weitere ionische oder nicht-ionische Emulgatoren oder deren Mischungen, wie eine Kombination aus nicht-ionischen und kationischen Emulgatoren, mit einem HLB-Wert von jeweils kleiner als 15
eingesetzt werden.

Beispiele für anionische Emulgatoren sind:

1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 30 Ethylenoxid (EO)- bzw. Propylenoxid(PO) einheiten.
2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 30 EO-Einheiten ethoxyliert sein.
3. Alkali- und Ammoniumsalze von Carbonsäuren mit 8 bis 20 C-Atomen im Alkyl-, Aryl-, Alkaryl- oder Aralkylrest.
4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 30 EO-Einheiten.

Beispiele für nichtionische Emulgatoren sind:

5. Polyvinylalkohol, der noch 5 bis 50%, vorzugsweise 8 bis 20% Vinylacetateinheiten aufweist, mit einem Polymerisationsgrad von 500 bis 3000.
6. Alkylpolyglycolether, vorzugsweise solche mit 3 bis 30 EO-Einheiten und Alkylresten von 8 bis 20 C-Atomen.
7. Alkylarylpolyglycolether, vorzugsweise solche mit 5 bis 30 EO-Einheiten und 8 bis 20 C-Atomen in den Alkyl- und Arylresten.
8. Ethylenoxid/Propylenoxid(EO/PO)-Blockcopolymere, vorzugsweise solche mit 8 bis 30 EO- bzw. PO-Einheiten.
9. Additionsprodukte von Alkylaminen mit Alkylresten von 8 bis 22 C-Atomen mit Ethylenoxid oder Propylenoxid.
10. Fettsäuren mit 6 bis 24 C-Atomen.
11. Alkylpolyglykoside der allgemeinen Formel $R^*-O-Z_O$, worin R* einen linearen oder verzweigten, gesättigten oder ungesättigten Alkylrest mit im Mittel 8-24 C-Atomen und $Z_O$ einen Oligoglykosidrest mit im Mittel o = 1-10 Hexose- oder Pentoseeinheiten oder Gemischen davon bedeuten.
12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen. 13. Polare Gruppen, enthaltend insbesondere die Elemente O, N, C, S, P, Si, enthaltende lineare Organo(poly)siloxane, insbesondere solche mit Alkoxygruppen mit bis zu 24 C-Atomen und/oder bis zu 40 EO- und/oder PO-Gruppen.

Beispiele für kationische Emulgatoren sind:

14. Salze von primären, sekundären und tertiären Fettaminen mit 8 bis 24 C-Atomen mit Essigsäure, Schwefelsäure, Salzsäure und Phosphorsäuren.

15. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

16. Quaternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate

Beispiele für ampholytische Emulgatoren sind:

17. Langkettig substituierte Aminosäuren, wie N-Alkyl-di-(aminoethyl-)glycin oder N-Alkyl-2-aminopropionsäuresalze.
18. Betaine, wie N-(3-Acylamidopropyl)-N,N-dimethylammoniumsalze mit einem $C_8$-$C_{18}$-Acylrest und Alkylimidazolium-Betaine.

Bevorzugt als Emulgatoren sind nichtionische Emulgatoren, insbesondere die vorstehend unter 6. aufgeführten Alkylpolyglycolether. Die Emulgatoren (D) können allein oder in Form eines Gemischs zweier oder mehrerer o.g. Emulgatoren verwendet werden, wobei sie in reiner Form oder als Lösungen eines oder mehrerer Emulgatoren in Wasser oder organischen Lösungsmitteln eingesetzt werden können.
Die erfindungsgemäßen Emulsionen enthalten Emulgatoren (D) vorzugsweise in Mengen von mindestens 0,5 Gew.-%, bevorzugt mindestens 1 Gew.-%, insbesondere mindestens 2 Gew.-%, und vorzugsweise höchstens 20 Gew.-%, bevorzugt höchstens 15 Gew.-%, insbesondere höchstens 10 Gew.-%.
Als weitere Inhaltsstoffe in den erfindungsgemäßen Emulsionen können
(E) nichtwässrige Lösemittel oder Co-Emulgatoren eingesetzt werden.
Die erfindungsgemäßen Emulsionen enthalten
(E) nichtwässrige Lösemittel oder Co-Emulgatoren in einer Menge von vorzugsweise mindestens 0,1 Gew.-%, bevorzugt mindestens 0,4 Gew.-%, insbesondere mindestens 0,8 Gew.-%, und vorzugsweise höchstens 20 Gew.-%, bevorzugt höchstens 15 Gew.-%, insbesondere höchstens 10 Gew.-%.
Die nichtwässrigen Lösungsmittel (E), die in den erfindungsgemäßen wässrigen Emulsionen eingesetzt werden können, stammen beispielsweise aus der Gruppe der ein- oder mehrwertigen Alkohole, Alkanolamine oder Glycolether. Beispiele für Lösungsmittel sind Ethanol, n- oder i-Propanol, Butanolen, wie 1-Butanol, 2-Butanol oder 2-Methyl-2-propanol, Pentanole, wie 1-Pentanol, 2-Pentanol oder 3-Pentanol, Hexanole, wie 1-Hexanol, 2-Hexanol oder 3-Hexanol, Heptanole, wie 1-Heptanol, 2-Heptanol, 3-Heptanol oder 4-Heptanol, Octanole, wie 1-Octanol, 2-Octanol, 3-Octanol oder 4-Octanol, Glycol, Propandiol, Butandiole, wie 1,2-Butandiol oder 1,3-Butandiol, Hexandiole, wie 1,2-Hexandiol oder 2-Methylpentan-2,4-diol, Octandiole, wie 2-Ethylhexan-1,3-diol oder 1,2-Octandiol, Glycerin, Diglykol, Propyl- oder Butyldiglykol, Ethylenglykolmethylether, Ethylenglykolethylether, Ethylenglykolpropylether, Ethylenglykolmono-n-butylether, Diethylenglykolmethylether, Diethylenglykolethylether, Diethylenglykolmono-n-butylether, Propylenglykolmethylether, Propylenglykolethylether, Propylenglykolpropylether, Propylenglykol-b-Butylether Propylen-glykol-tert-butylether, Methoxytriglykol, Ethoxytriglykol, Butoxytriglykol, Dipropylenglykolmonomethylether, Dipropylenglykolmonoethylether, 1-Butoxyethoxy-2-propanol oder 3-Methyl-3-methoxy-butanol, 1-Aminobutan, 2-Aminobutan, 2-Amino-2-methylpropan, 1-Aminopentan, 2-Aminopentan, 1-Aminohexan, 1-Aminoheptan und 1-Aminooctan; Ethyl-, Propyl-, Isopropyl-, Butyl-, Isobutyl-, Pentyl-, Isopentyl- und Hexylacetat; Methyl-, Ethyl- und tert.-Butylpropionat; Methyl-, Ethyl-, Propyl- und Butylbutyrat; 2-Butanon, 2-Pentanon, 3-Pentanon, 4-Methyl-2-pentanon, 2-Hexanon, 3-Hexanon, 2-Heptanon, 3-Heptanon, 4-Heptanon, 5-Methyl-3-Heptanon, 2-Octanon und 3-Octanon sowie Mischungen dieser Cotenside.
Beispiele für bevorzugte nichtwässrige Lösungsmittel oder Co-Emulgatoren (E) sind 1-Alkanole der vorstehend aufgeführten Beispiele mit $C_5$- bis $C_8$-Ketten, Alkandiole der vorstehend aufgeführten Beispiele mit $C_4$- bis $C_8$-Ketten, Glycerin, Propyl-, Butyl- und Pentylacetat, 2-Pentanon sowie die oben aufgeführten Ethylenglykol-, Propylenglykol-, Dipropylenglykol- oder Diethylenglykolmonoalkylether.
Als nichtwässrige Lösungsmittel oder Co-Emulgatoren (E) besonders bevorzugt sind 1-Pentanol, 1-Hexanol, 1-Octanol, Propandiol, 1,3-Butandiol, 1,2-Hexandiol, 2-Ethylhexan-1,3-diol, 1,2-Octandiol, Glycerin, Diethylenglykolmethylether, Diethylenglykolethylether, Diethylenglykolmono-n-butylether, Propylenglykolmethylether.
Ferner können Polyalkylenglykole, wie Polyethylenglykole (z.B. PEG600, PEG1000 oder PEG6000) oder Polypropylenglykole (z.B. PPG2000), Polyalkylenblockpolymere mit einem HLB-Wert von kleiner 15, wie die sogenannten Poloxamere (Blockcopolymere aus Ethylenoxid- und Propylenoxid-Einheiten), wie z.B. PEG-PPG-PEG-Blockpolymer Pluronic® L-31, PEG-PPG-PEG-Blockpolymer Pluronic® L-61, PPG-PEG-PPG-Blockpolymer Pluronic® 17R4, PPG-PEG-PPG Pluronic®-Blockpolymer 31R1 (HLB jeweils < 7) (erhältlich bei Sigma-Aldrich) oder auch Poloxamine (Copolymere aus Ethylenoxid- und Propylenoxid-Einheiten, die über einen Ethylendiamin-Kern verbrückt sind) mit einem HLB-Wert von kleiner 15, wie z.B. Tetronic 701 oder Tetronic 90R4 (HLB jeweils < 7) (erhältlich bei Sigma-Aldrich) als Co-Emulgatoren verwendet werden.

Als weitere Inhaltsstoffe in den erfindungsgemäßen Emulsionen können
(F) Hilfsstoffe, wie pH-Regulierungsmittel, Salze, Schauminhibitoren, Verdicker und/oder Schutzkolloide, Konservierungsmittel, Desinfektionsmittel, Netzmittel, Korrosionsinhibitoren, Farbstoffe, Duftstoffe oder deren Mischungen, eingesetzt werden.

Als pH-Regulierungsmittel einsetzbar sind hier sämtliche bekannte Säuren bzw. Laugen, sofern sich ihr Einsatz nicht aus anwendungstechnischen oder ökologischen Gründen bzw. aus Gründen des Verbraucherschutzes verbietet.

Die eingesetzten Säuren dienen dabei zur Einstellung eines gewünschten pH-Werts oder können Säureadditionssalze mit den Amino-haltigen Resten (Y) der Carbamato-funktionalisierten Polydiorganosiloxane (A) ausbilden.

Beispiele für Mineralsäuren, die sich beispielsweise mit den vorstehend genannten Amino-haltigen Resten (Y) umsetzen lassen, sind Salzsäure, Perchlorsäure, Schwefelsäure, schweflige Säure, Salpetersäure, salpetrige Säure, Flusssäure, Phosphor-, Diphosphor- und Polyphosphorsäuren. Beispiele für geeignete Carbonsäuren sind Ameisensäure, Essigsäure, Propionsäure, Butansäuren, Citronensäure, Trichloressigsäure, Dichloressigsäure und Chloressigsäure, Trifluoressigsäure, Cyanessigsäure, Phenylessigsäure, Benzoesäure, m- und p-Nitrobenzoesäure, Oxalsäure, Malonsäure und Milchsäure.

Besonders bevorzugt sind Essigsäure und Ameisensäure.

Im Rahmen der vorliegenden Erfindung wird der pH-Wert mit einer Elektrode entsprechend der US Pharmacopeia USP 33 bei 20°C gemessen.

Beispiele für Salze (Elektrolyte) sind insbesondere solche aus der Gruppe der anorganischen Salze, wobei eine breite Anzahl der verschiedensten Salze eingesetzt werden kann. Bevorzugte Kationen sind die Alkali- und Erdalkalimetalle, bevorzugte Anionen sind die Halogenide und Sulfate. Aus herstellungstechnischer Sicht ist der Einsatz von Natriumacetat oder Natriumchlorid in den erfindungsgemäßen wässrigen Emulsionen bevorzugt.

Beispiele für Schauminhibitoren sind Seifen, Paraffine oder Silikonöle.

Beispiele für Konservierungsmittel sind Methylisothiazolinon, Chlormethylisothiazolinon, Benzylisothiazolinon, Phenoxyethanol, Methylparaben, Ethylparaben, Propylparaben, Butylparaben, Isobutylparaben, Alkalibenzoate, Alkalisorbate, Iodopropynylbutylcarbamat, Benzylalkohol und 2-Brom-2-nitropropan-1,3-diol.

Die Emulgierung der erfindungsgemäßen Carbamato-funktionalisierten Polydiorganosiloxane (A) mit Hilfe der erfindungsgemäßen nicht-ionischen, Polyethylenoxid-haltigen Emulgatoren (B) erfolgt dadurch, dass die Carbamato-funktionalisierten Polydiorganosiloxane (A) in Wasser (C) mit den Emulgatoren (B), gegebenenfalls den Emulgatoren (D), gegebenenfalls dem nichtwässrigen Lösemittel oder Co-Emulgatoren (E), gegebenenfalls den weiteren Hilfsstoffen (F) miteinander intensiv gemischt werden. Es werden stabile Emulsionen gebildet, in denen die Carbamato-funktionalisierten Polydiorganosiloxane (A) in fein verteilter Form vorliegen.

Der Emulgiervorgang zur Herstellung der erfindungsgemäßen wässrigen Emulsionen von Carbamato-funktionalisierten Polydiorganosiloxanen (A) wird vorzugsweise bei Temperaturen von mindestens 10°C, bevorzugt mindestens 15°C und vorzugsweise höchstens 80°C, bevorzugt höchstens 70°C, durchgeführt.

Die Temperaturerhöhung kommt vorzugsweise durch den Eintrag mechanischer Scherenergie, die für den Emulgierprozess benötigt wird, zustande. Die Temperaturerhöhung wird nicht zur Beschleunigung eines chemischen Prozesses benötigt. Weiterhin wird das erfindungsgemäße Verfahren vorzugsweise beim Druck der umgebenden Atmosphäre durchgeführt, kann aber auch bei höheren oder niederen Drücken durchgeführt werden.

Die Herstellung kann diskontinuierlich oder kontinuierlich erfolgen.

Technologien zur Herstellung von Emulsionen von Organopolysiloxanen sind bekannt. So kann das intensive Mischen und Dispergieren in Rotor-Stator-Rührvorrichtungen, Kolloidmühlen, Hochdruckhomogenisatoren, Mikrokanälen, Membranen, Strahldüsen und ähnlichem, oder mittels Ultraschall erfolgen. Homogenisiergeräte und Verfahren sind z.B. in Ullmann's Encyclopedia of Industrial Chemistry, CD-ROM-Ausgabe 2003, Wiley-VCH Verlag, unter dem Stichwort "Emulsions" beschrieben.

Die Art der Mischung der Komponenten, die zur Herstellung der erfindungsgemäßen Emulsionen gebraucht werden, ist nicht sehr kritisch und kann in verschiedener Reihenfolge ausgeübt werden. In Abhängigkeit von den Komponenten (A), (B), gegebenenfalls (D), gegebenenfalls (E) und gegebenenfalls (F) können sich aber bevorzugte Vorgehensweisen ergeben, die im Einzelfall geprüft werden sollten.

Es können z.B. die Komponente (A) und gegebenenfalls eine Säure (F') vorgelegt werden, daraufhin die Emulgatoren (B) und gegebenenfalls (D) und gegebenenfalls die Coemulgatoren (E) zugefügt und danach das Dispersionsmittel Wasser (C) und gegebenenfalls weitere Hilfsmittel (F) eingearbeitet werden. In vielen Fällen hat sich gezeigt, dass es vorteilhaft ist, Emulgatoren (B), gegebenenfalls Emulgatoren (D) und gegebenenfalls Coemulgatoren (E) und gegebenenfalls eine Säure (F') als Hilfsmittel mit einem Teil des Dispersionsmittels Wasser (C) in der Emulgierapparatur vorzulegen und in diese erhaltene Mischung Komponente (A) und die weiteren Komponenten einzuarbeiten.

Ein weiterer Gegenstand der Erfindung sind kosmetische Zusammensetzungen enthaltend
in einem kosmetisch akzeptablen Medium, vorzugsweise Wasser, mindestens ein Haar-Konditionierungsmittel und mindestens eine erfindungsgemäße wässrige Emulsion.

Ein weiterer Gegenstand der Erfindung ist ein Verfahren zur Behandlung von keratinischen Fasern, bevorzugt Haaren, mit den erfindungsgemäßen kosmetischen Zusammensetzungen.

Vorzugsweise handelt es sich bei diesen Zusammensetzungen um kosmetische Zusammensetzungen, die die Reinigung und Pflege von Haaren zur Aufgabe haben. Beispiele für Mittel zur Reinigung und Pflege von Haaren sind Haar-Shampoos, -Spülungen (Rinse-Off Conditioner), -Kuren, -Masken, -Seren, Schäume, -Stylingsprays, -Cremes, -Gele, -öle, -Spitzenfluide und -Färbemittel.

Pflegen in diesem Zusammenhang bedeutet, die keratinischen Fasern in deren ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen), wie beispielsweise Altern, Verschmutzen, Ausbleichen, zu mindern oder zu vermeiden oder sogar gewünschte positive Eigenschaften keratinischer Fasern zu verbessern. Beispiele hierzu sind die Verbesserung von Weichheit und Glätte, die Reduzierung von Kämmkräften, Glanzeigenschaften, die Verbesserung von Farbeindrücken, Farbschutzeigenschaften, Verringerung der elektrostatischen Aufladungen, Schutzeigenschaften bei thermischer Belastung des Haars oder Hydrophobisierung.

Die erfindungsgemäßen wässrigen Emulsionen werden in diesen Zusammensetzungen vorzugsweise in Mengen von 0,1 bis 40 Gew.%, bevorzugt von 0,2 bis 30 Gew.%, besonders bevorzugt 0,4 bis 20 Gew.%, jeweils bezogen auf das Gesamtgewicht der Zusammensetzungen, eingesetzt.

Die Zusammensetzung, in der die erfindungsgemäßen wässrigen Emulsionen eingesetzt werden, kann vorliegen in Form einer W/O-Emulsion (Wasser-in-Öl-Emulsion), einer O/W-Emulsion (Öl-in-Wasser-Emulsion) oder als multiple Emulsion.

Bevorzugt ist in der kosmetischen Zusammensetzung das kosmetisch akzeptable Medium Wasser.

Bei den Zusammensetzungen, in der die erfindungsgemäßen wässrigen Emulsionen eingesetzt werden, handelt es sich bevorzugt um Zusammensetzungen mit transluzenter oder transparenter Erscheinungsform.

Bei diesen bevorzugten Zusammensetzungen handelt es sich um transparente bzw. transluzente Mikroemulsionen mit Tröpfchendurchmesser im Bereich von etwa 0,01 μm bis etwa 0,1 μm.
Dagegen liegen in einfachen Emulsionen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 μm bis ca. 50 μm. Solche "Makroemulsionen" sind, ohne weitere färbende Zusätze, milchig weiß gefärbt und opak. Feinere "Makroemulsionen", deren Tröpfchendurchmesser im Bereich von ca. 0,1 μm bis ca. 1 μm liegen, sind, wiederum ohne färbende Zusätze, bläulich weiß gefärbt und undurchsichtig.
Mizellaren und molekularen Lösungen mit Partikeldurchmessern kleiner als ca. 0,01 μm, ist vorbehalten, klar und transparent zu erscheinen.

Die kosmetischen Zusammensetzungen, in der die erfindungsgemäßen wässrigen Emulsionen eingesetzt werden, enthalten mindestens ein Haar-Konditionierungsmittel, im Folgenden einfach als Konditionierungsmittel genannt. Als Konditionierungsmittel werden analog zu K. Krummel, Stephane Chiron, J. Jachowicz, Chapter 14, in: "The Chemistry and Manufacture of Cosmetics", Volume II, Formulating, Third Edition by Mitchell L. Schlossmann, 2000, S. 359-396, kosmetische Inhaltsstoffe bezeichnet, welche die Haaroberfläche modifizieren und die Beschaffenheit des Haares beeinflussen. Kosmetische Zusammensetzungen enthaltend Konditionierungsmittel werden eingesetzt zur Modifizierung oder Verbesserung der Weichheit der Haare, besseren Entwirrbarkeit, Verringerung der Nass- und Trockenkämmkraft, Pflege der Haare, Vermeidung elektrostatischer Aufladung, leichterer Gleitwirkung durch das Haar und entlang der Haaroberfläche, Verbesserung des Haarglanzes, Erhalt der Farbechtheit von Haaren, Verringerung des Haarbruchs, Erhalt der Haarform und weiteren kosmetischen Eigenschaften, die mit natürlichem und gesundem Haar in Verbindung gebracht werden.

Die Zusammensetzung, in der die erfindungsgemäßen wässrigen Emulsionen eingesetzt werden, verbessert einen oder mehrere der oben genannten Effekte.

Beispiele für Konditionierungsmittel und deren INCI-Namen sind beschrieben im "International Cosmetic Ingredient Dictionary and Handbook" des Personal Care Product Council (Hrsg.). Als Referenz kann das World Wide Web basierte "wINCI" Web Based International Cosmetic Ingredient Dictionary and Handbook (http://online.personalcarecouncil.org/jsp/Home.jsp) oder das International Cosmetic Ingredient Dictionary and Handbook, 13th Edition, The Personal Care Products Council (formerly: The Cosmetic, Toiletry, and Fragrance Association (CTFA)), 2010, Verwendung finden.

Bevorzugte Beispiele für Konditionierungsmittel sind kationische Polymere. Darunter verstanden werden Polymere, die seitenständig oder endständig kationische Gruppen tragen oder seitenständig oder endständig Gruppen, die durch Ionisierung in eine kationische Gruppe überführt werden können. Vorzugsweise werden kationische Polymere verwendet, die eine quaternäre Ammoniumgruppe aufweisen.

Beispiele für vorzugsweise eingesetzte kationische Polymere sind im International Cosmetic Ingredient Dictionary and Handbook unter der Bezeichnung Polyquaternium veröffentlicht, wobei jedes Polymer mit einem individuellen Zahlenkürzel identifiziert wird, z. B. Polyquaternium-1.

Weitere Beispiele für kationische Polymere sind quaternäre Ammoniumgruppen aufweisende Derivate von modifizierten Polysacchariden, z. B. Polymere mit den INCI-Namen Cassia Hydroxypropyltrimonium Chloride, Derivate von modifizierter Cellulose und/oder Stärke, z. B. ein quaternäres Ammoniumderivat eines mit Propylenglykolether modifizierten Cyamopsis Tetragonoloba (Guar) Gums mit dem INCI-Namen Guar Hydroxypropyltrimonium Chloride, oder polymere quaternäre Ammoniumsalze des Reaktionsprodukts von Hydroxyethylcellulose mit einem Trimethylammonium-substituierten Epoxid, wie Cellulose, 2-hydroxyethyl 2-(2-hydroxy-3-(trimethylammonium)propoxy)ethyl 2-hydroxy-3-(trimethylammonium)propyl ether chlorid, wie Cellulose, 2-hydroxyethyl 2-hydroxy-3-(trimethylammonium)propyl ether, chlorid, wie Cellulose, 2-hydroxyethyl 3-hydroxy-3 (trimethylammonium)propyl ether, chloride, wie Cellulose, 2-[2-hydroxy-3-(trimethylammonium)propoxy]ethyl ether, chloride, mit dem INCI-Namen Polyquaternium-10.

Weitere Beispiele für kationische Polymere sind quaternäre Ammoniumgruppen aufweisende Acrylsäurepolymerderivate, Acrylsäurecopolymerderivate, Methacrylsäurederivate und Methacrylsäurecopolymerderivate, z. B. Polymere mit dem INCI-Namen Polyquaternium-37.

Weitere Beispiele für kationische Polymere sind quaternäre Ammoniumgruppen aufweisende Copolymere aus Dimethyldiallylammoniumchlorid und Acrylsäure, z. B. Polymere mit dem INCI-Namen Polyquaternium-22.

Weitere Beispiele für kationische Polymere sind quaternäre Ammoniumgruppen aufweisende Copolymere aus Derivaten des Vinylpyrrolidon, Vinylimidazol und Vinylimidazolin und Methacrylsäure, z. B. Polymere mit dem INCI-Namen Polyquaternium-86.

Weitere Beispiele für kationische Polymere sind quaternäre Ammoniumgruppen aufweisende Copolymere aus Acrylamid und Dimethyl-diallyl-ammoniumchlorid, z. B. Polymere mit dem INCI-Namen Polyquaternium-7.

Weitere Beispiele für kationische Polymere sind quaternäre Ammoniumgruppen aufweisende Copolymere aus dem Reaktionsprodukt von Diethylsulfat mit Vinylpyrrolidon und Dimethylaminoethylmethacrylat, z. B. Polymere mit dem INCI-Namen Polyquaternium-11.

Die Zusammensetzung, in der die erfindungsgemäßen wässrigen Emulsionen eingesetzt werden, enthält kationische Polymere vorzugsweise in Mengen von 0,01 bis 5 Gew.-%, bevorzugt von 0,05 bis 4 Gew.-%, insbesondere bevorzugt 0,10 bis 3 Gew.-%, jeweils bezogen auf das Gesamtgewicht der Zusammensetzung.

Weitere bevorzugte Beispiele für Konditionierungsmittel sind kationische Tenside. Beispiele für vorzugsweise eingesetzte kationische Tenside entsprechen den in Punkt 14. bis 16. unter Beispiele für kationische Emulgatoren aufgeführten Materialien. Beispiele sind Cetyltrimethylammoniumsalze oder Behenyltrimethylammoniumsalze. Als anionisches Gegenion können beispielsweise Chlorid, Bromid, Methosulfat vorliegen. INCI-Namen von vorzugsweise eingesetzten kationischen Tensiden sind beispielsweise Cetrimonium Chloride, Cetrimonium Methosulfate, Behentrimonium Chloride, Behentrimonium Methosulfate, Steartrimonium Bromide.

Die Zusammensetzung, in der die erfindungsgemäßen wässrigen Emulsionen eingesetzt werden, enthält kationische Tenside vorzugsweise in Mengen von 0,1 bis 7 Gew.-%, bevorzugt von 0,15 bis 6 Gew.-%, insbesondere bevorzugt 0,2 bis 5 Gew.-%, jeweils bezogen auf das Gesamtgewicht der Zusammensetzung.

Weitere Beispiele für Konditionierungsmittel sind nicht polymer vorliegende quaternäre Ammoniumverbindungen. Darunter verstanden werden nicht polymere Ammoniumverbindungen, die kationisch vorliegen oder durch Ionisierung in eine kationische Gruppe überführt werden können. Beispiele für vorzugsweise eingesetzte nicht polymer vorliegende quaternäre Ammoniumverbindungen sind Dimethyl Dioctadecyl Ammonium Chlorid mit dem INCI-Namen Distearyldimonium Chloride, N-[3-(Dimethylamino)propyl]octadecanamid mit dem INCI-Namen Stearamidopropyl Dimethylamine oder Verbindungen mit dem INCI-Namen Dicocoylethyl Hydroxyethylmonium Methosulfate oder Quaternium-87.

Weitere bevorzugte Beispiele für Konditionierungsmittel sind Organopolysiloxane und Organopolysiloxancopolymere, die von den in den erfindungsgemäßen wässrigen Emulsionen vorliegenden Carbamato-funktionalisierten Polydiorganosiloxanen (A) verschieden sind. Die Organopolysiloxane können in Form eines Öls, Wachses, Gummis oder Harzes vorliegen oder in Form einer Emulsion.

Beispiele für solche von Carbamato-funktionalisierten Polydiorganosiloxanen (A) verschiedenen Organopolysiloxane sind:
Cyclische Organopolysiloxane der Formel $[R^*{}_2SiO]_x$,
wobei x gleich eine ganze Zahl von 4 bis 8 ist,
lineare Organopolysiloxane der allgemeinen Formel

$$R^*{}_3SiO[R^*{}_2SiO]_ySiR^*{}_3$$

oder

$$HOSiR^*{}_2O[R^*{}_2SiO]_ySiR^*{}_2OH,$$

wobei y gleich 0 oder eine ganze Zahl von 1 bis 2000 ist, und
harzartige Organopolysiloxane der allgemeinen Formel $R^*_tSiO_{(4-t)/2}$ wobei R* jeweils die oben dafür angegebene Bedeutung von $R^1$ oder Y' hat und t gleich 0, 1, 2 oder 3 ist,
so dass das Organopolysiloxanharz aus M, D, T und/oder Q-Einheiten aufgebaut ist, wobei die Kombination vorwiegend oder ausschließlich aus D und T-Einheiten ebenso bevorzugt sind, wie die Kombination vorwiegend oder ausschließlich aus M und Q-Einheiten, wobei im Falle der vorwiegend oder ausschließlich aus D und T-Einheiten aufgebauten Harze T-Einheiten bevorzugt in einem Molverhältnis von T/[M + D + T + Q] von 0,45 bis 1, besonders bevorzugt von 0,55 bis 1,0 vorliegen und die Anzahl der M und Q-Einheiten in beiden Fällen vorzugsweise Null ist und in dem Fall der vorwiegend oder ausschließlich aus M und Q-Einheiten aufgebauten Organopolysiloxanharze Q-Einheiten bevorzugt in einem Molverhältnis von Q/[M + D + T + Q] von 0,25 bis 0,9, besonders bevorzugt von 0,35 bis 0,7 vorliegen und die Anzahl der D und T-Einheiten in beiden Fällen vorzugsweise Null ist.

Beispiele für Organopolysiloxane, vorliegend in Form eines Öls sind Polydimethylsiloxane mit der Viskosität 0,65 bis 2000000 mPas (25°C) und den INCI-Bezeichnungen Disiloxan und Dimethicone.

Weitere Beispiele für Organopolysiloxane, vorliegend in Form eines Öls oder Wachses sind funktionalisierte Organopolysiloxane, beispielsweise Polyalkylsiloxane, wobei mindestens ein Alkylrest sich unterscheidet von Methyl, beispielsweise Organopolysiloxane mit dem INCI-Namen Stearyl Dimethicone, Cetyl Dimethicone oder C26-28 Alkyl Dimethicone, oder beispielsweise Polyarylsiloxane und Polyarylalkylsiloxane, beispielsweise Organopolysiloxane mit dem INCI-Namen Phenyl Trimethicone, Trimethylsiloxyphenyl Dimethicone oder Dimethylphenyl Dimethicone, oder beispielsweise Organopolysiloxane mit einem organofunktionellen Rest wie einem Aminopropyl-, Aminopropyl-aminoethyl, Aminopropylaminoisobutylrest, beispielsweise Organopolysiloxane mit dem INCI-Namen Amodimethicone, oder beispielsweise Organopolysiloxane mit einem Polyethylenglycol- oder Polyalkylenglycolrest, beispielsweise Organopolysiloxane mit dem INCI-Namen PEG-12 Dimethicone, PEG/PPG-25,25-Dimethicone oder Cetyl PEG/PPG-15/15 Butyl Ether Dimethicone.

Weitere Beispiele für Organopolysiloxane sind Siliconharze mit den INCI-Namen Trimethylsiloxysilicate oder Polymethylsilsesquioxane.

Die Zusammensetzung, in der die erfindungsgemäßen wässrigen Emulsionen eingesetzt werden, enthält Organopolysiloxane und Organopolysiloxancopolymere, die von den Carbamato-funktionalisierten Polydiorganosiloxanen (A) verschieden sind, vorzugsweise in Mengen von 0,1 bis 40 Gew.-%, bevorzugt von 0,2 bis 30 Gew.-%, insbesondere bevorzugt 0,3 bis 20 Gew.-%, jeweils bezogen auf das Gesamtgewicht der Zusammensetzung.

Weitere bevorzugte Beispiele für Konditionierungsmittel sind Fettsäureester und Fettsäurealkohole.

Beispiele für Fettsäurealkohole sind Alkohole mit $C_8$-$C_{28}$ Kohlenstoffketten wie die Fettalkohole 1-Octadecanol mit dem INCI-Namen Stearyl Alcohol, 1-Hexadecanol mit dem INCI-Namen Cetyl Alcohol, oder Fettalkohole mit den INCI-Namen Cetearyl Alcohol, Myristyl Alcohol, Caprylic Alcohol, Lauryl Alcohol, Decyl Alcohol und Oleyl Alcohol.

Fettsäurealkohole erfüllen neben konditionierenden Eigenschaften auch eine strukturgebende, verdickende Wirkung bei kosmetischen Zusammensetzungen.

Weitere Beispiele für Fettsäureester sind Ester der Fettsäuren mit den INCI-Namen Palmitic Acid, Oleic Acid, Linolic Acid, Linoleic Acid, Caprylic Acid, Myristic Acid, Stearic Acid, beispielsweise Fettsäureester mit den INCI-Namen Isopropyl Palmitate, Ethylhexyl Palmitate, Isopropyl Myristate, Isopropyl Stearate.

Die Zusammensetzung, in der die erfindungsgemäßen wässrigen Emulsionen eingesetzt werden, enthält Fettsäureester und Fettsäurealkohole vorzugsweise in Mengen von 0,1 bis 15 Gew.-%, bevorzugt von 0,3 bis 12 Gew.-%, insbesondere bevorzugt 0,5 bis 10 Gew.-%, jeweils bezogen auf das Gesamtgewicht der Zusammensetzung.

Weitere bevorzugte Beispiele für Konditionierungsmittel sind natürliche oder synthetische Öle und Wachse.

Beispiele für bevorzugte Öle und Wachse sind Kohlenwasserstoffe mit linearen oder verzweigten, gesättigten oder ungesättigten $C_4$-$C_{60}$ Kohlenstoffketten, wie Öle und Wachse mit den INCI-Namen Isododecane, hydrated Polyisobutylene, hydrated Polydecene, Paraffin und Isoparaffin.

Weitere Beispiele für bevorzugte Öle und Wachse sind Carnaubawachs, Bienenwachs, Wollwachs, mikrokristallines Wachs, Jojobaöl, Reisöl, Calendulaöl, Sonnenblumenöl, Sojaöl, Kokosnussöl, Olivenöl und Mandelöl.

Die Zusammensetzung, in der die erfindungsgemäßen wässrigen Emulsionen eingesetzt werden, enthält Öle und Wachse vorzugsweise in Mengen von 0,1 bis 10 Gew.-%, bevorzugt von 0,2 bis 7 Gew.-%, insbesondere bevorzugt 0,3 bis 5 Gew.-%, jeweils bezogen auf das Gesamtgewicht der Zusammensetzung.

Weitere bevorzugte Beispiele für Konditionierungsmittel sind Panthenol, Lipide, wie Ceramide, Proteine und hydrolysierte Proteine, wie hydrolisiertes Kollagen, hydrolysierte Weizenproteine und hydrolysierte Seide.

Als Haar-Konditionierungsmittel werden bevorzugt solche ausgewählt aus der Gruppe von
kationischen Polymeren,
kationischen Tensiden,
nicht polymer vorliegenden quaternären Ammoniumverbindungen, Organopolysiloxanen und Organopolysiloxancopolymeren, die von den in den erfindungsgemäßen wässrigen Emulsionen vorliegenden Carbamato-funktionalisierten

Polydiorganosiloxanen (A) verschieden sind,
Fettsäureestern und Fettsäurealkoholen,
natürlichen oder synthetischen Ölen und Wachsen und Panthenol, Lipiden, Proteinen und hydrolysierten Proteinen, sowie deren Mischungen
eingesetzt.

Optional enthält die Zusammensetzung weitere kosmetisch übliche Additive, wie z. B. Tenside, Verdicker, Gelierungsmittel, Filmbildner, feuchtigkeitsspendende Mittel, UV-Filter, Perlglanzpigmente, Vitamins, Antioxidantien, Coffein, Anti-Schuppenwirkstoffe oder Konservierungsmittel oder deren Mischungen.

Beispiele für weitere in der Kosmetik übliche Additive und deren INCI-Namen sind beschrieben im "International Cosmetic Ingredient Dictionary and Handbook" des Personal Care Product Council.

Optional enthält die Zusammensetzung weitere kosmetisch übliche Additive wie Tenside.

Beispiele für in der Kosmetik übliche Tenside sind auch in K. Schrader, A. Domsch, Cosmetology - Theory and Practice, Volume II, Seite II-8 bis II-22, Verlag für chemische Industrie, 2005, sowie in Punkt 1. bis 18. unter Beispiele für Emulgatoren, beschrieben.

Beispiele für vorzugsweise eingesetzte anionische Tenside entsprechen den in Punkt 1. bis 3. unter Beispiele für anionische Emulgatoren aufgeführten Materialien.

INCI-Namen von vorzugsweise eingesetzten anionische Tensiden sind beispielsweise Sodium Lauryl Sulfate, Ammonium Laureth Sulfate, Sodium Laureth Sulfate, Disodium 2-Sulfolaurate, Disodium Lauryl Sulfosuccinate oder Disodium Laureth-Sulfosuccinate.

Die Zusammensetzung, in der die erfindungsgemäßen wässrigen Emulsionen eingesetzt werden, enthält anionische Tenside vorzugsweise in Mengen von 1 bis 30 Gew.-%, bevorzugt von 5 bis 25 Gew.-%, insbesondere bevorzugt 7 bis 20 Gew.-% jeweils bezogen auf das Gesamtgewicht der Zusammensetzung.

Beispiele für vorzugsweise eingesetzte nichtionische Tenside entsprechen den in Punkt 5. bis 13. unter Beispiele für nichtionische Emulgatoren aufgeführten Materialien. INCI-Namen von vorzugsweise eingesetzten nichtionischen Tensiden sind beispielsweise Coco Glucoside, Lauryl glucoside, Decyl Glucoside, PEG-40 Hydrogenated Castor Oil, Polysorbate 80 oder PEG-7 Glyceryl Cocoate.

Die Zusammensetzung, in der die erfindungsgemäßen wässrigen Emulsionen eingesetzt werden, enthält nichtionische Tenside vorzugsweise in Mengen von 1 bis 15 Gew.-% bevorzugt von 2 bis 12 Gew.-%, insbesondere bevorzugt 3 bis 10 Gew.-%, jeweils bezogen auf das Gesamtgewicht der Zusammensetzung.

Beispiele für vorzugsweise eingesetzte amphotere Tenside entsprechen den in Punkt 17. bis 18. unter Beispiele für nichtionische Emulgatoren aufgeführten Materialien. Weitere bevorzugte Beispiele sind Verbindungen aus den Klassen der Alkylamidobetaine, Alkylamphoacetate und Alkylamphopropionate. INCI-Namen von vorzugsweise eingesetzten nichtionischen Tensiden sind beispielsweise Cocamidopropyl Betaine, Cetyl Betaine, Cocamide MEA, Cocamide DEA, Cocamide MIPA, Sodium Cocoamphoacetate und Sodium Cocoamphopropionate.

Die Zusammensetzung, in der die erfindungsgemäßen wässrigen Emulsionen eingesetzt werden, enthält amphotere Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der Zusammensetzung.

Optional enthält die Zusammensetzung weitere kosmetisch übliche Additive wie Verdicker.

Beispiele für vorzugsweise eingesetzte Verdicker sind modifizierte Polysaccharide wie Stärke, Cellulose, Gummi Arabicum und Guar Gums, z. B. Polymere mit dem INCI-Namen Cellulose Gum, Guar Gum, Xanthan Gum oder Cassia Gum.

Weitere Beispiele für Verdicker sind hydrophob modifizierte nichtionische Cellulosederivate, z. B. das Cellulosederivat mit dem INCI-Namen Hydroxyethylcellulose.

Weitere Beispiele für Verdicker sind vernetzte Acrylsäure- und Methacrylsäurepolymere und Derivate der vernetzten Acrylsäure- und Methacrylsäurepolymere, z. B. Polymere mit dem INCI-Namen Carbomer.

Weitere Beispiele für Verdicker sind Agentien, die in Kombination mit Tensiden eine verdickende Wirkung erzielen. Beispiele sind Monoglyceride von Fettsäuren, Mono/Diglyceride von ethoxylierten Fettsäuren und ethoxylierte Fettalkohole. INCI-Namen von vorzugsweise eingesetzten Verdickern die in Kombination mit Tensiden eine verdickende Wirkung erzielen sind PEG-120 Methyl Glucose Dioleate, PEG-150 Distearate, Myristyl Glycol, PEG-200 Glyceryl Palmitate, Laureth-4 oder PEG-200 Glyceryl Palmitate.

Weitere Beispiele für Verdicker sind Salze, z. B. Salze mit dem INCI-Namen Sodium Chloride.

Die Zusammensetzung, in der die erfindungsgemäßen wässrigen Emulsionen eingesetzt werden, enthält Verdicker vorzugsweise in Mengen von 0,1 bis 10 Gew.-%, jeweils bezogen auf das Gesamtgewicht der Zusammensetzung.

Optional enthält die Zusammensetzung weitere kosmetisch übliche Additive wie Filmbildner.

Bevorzugte Beispiele für Filmbildner sind Polymere.

Beispiele für vorzugsweise eingesetzte filmbildende Polymere sind beschrieben im "International Cosmetic Ingredient Dictionary and Handbook" des Personal Care Product Councils.

Beispiele für bevorzugte filmbildende Polymere sind Acrylsäurepolymerderivate, Acrylsäurecopolymerderivate,

Methacrylsäurederivate und Methacrylsäurecopolymerderivate. Beispiele für bevorzugte anionische Polymere sind Copolymere aus Vinyl Acetate und einem oder mehrerer Acrylsäure-, Methacrylsäuremonomere und deren Ester, z. B Polymere mit dem INCI-Namen Acrylates/VA Copolymer.

Weitere Beispiele für bevorzugte filmbildende Polymere sind Copolymere aus Vinylpyrrolidone und einem oder mehrere Acrylsäure-, Methacrylsäuremonomere und deren Ester, z. B. Polymere mit dem INCI-Namen Acrylates/VP Copolymer.

Weitere Beispiele für bevorzugte filmbildende Polymere sind Copolymere aus tert-Butyl Acrylamide und einem und einem oder mehrerer Acrylsäure-, Methacrylsäuremonomere und deren Ester, z. B. Polymere mit dem INCI-Namen Acrylates/t-Butylacrylamide Copolymer.

Weitere Beispiele für bevorzugte filmbildende Polymere sind Copolymere aus Vinyl Acetate, Crotonsäure und Vinyl Neodecanoate Monomeren, z. B. Polymere mit dem INCI-Namen VA/Crotonates/Vinyl Neodecanoate Copolymer.

Weitere Beispiele für bevorzugte filmbildende Polymere sind Copolymere aus Vinyl Acetate, Crotonsäure und Vinyl Neodecanoate Monomeren und Vinylsiliconen, z. B. Polymere mit dem INCI-Namen Crotonic Acid/Vinyl C8-C12 Isoalkyl Esters/VA/Bis-Vinyldimethicone Copolymer.

Die Zusammensetzung, in der die erfindungsgemäßen wässrigen Emulsionen eingesetzt werden, enthält filmbildende Polymere vorzugsweise in Mengen von 0,1 bis 15 Gew.-%, bevorzugt von 0,2 bis 10 Gew.-%, insbesondere bevorzugt 0,3 bis 7 Gew.-%, jeweils bezogen auf das Gesamtgewicht der Zusammensetzung.

Optional enthält die Zusammensetzung weitere kosmetisch übliche Additive wie feuchtigkeitsspendende Mittel.

Beispiele für vorzugsweise eingesetzte feuchtigkeitsspendende Mittel sind Glycerin, Sorbitol, Xylitol, Polyethylenglycol, 1,2-Propandiol, 1,3-Propandiol oder Polypropylenglykol.

Die Zusammensetzung, in der die erfindungsgemäßen wässrigen Emulsionen eingesetzt werden, enthält feuchtigkeitsspendende Mittel vorzugsweise in Mengen von 0,1 bis 10 Gew.-%, bevorzugt von 0,2 bis 8 Gew.-%, insbesondere bevorzugt 0,3 bis 6 Gew.-%, jeweils bezogen auf das Gesamtgewicht der Zusammensetzung.

Optional enthält die Zusammensetzung weitere kosmetisch übliche Additive wie perlglanzgebende Mittel.

Beispiele für vorzugsweise eingesetzte perlglanzgebende Mittel sind Perlglanzpigmente oder Glycol Distearate.

Die Zusammensetzung, in der die erfindungsgemäßen wässrigen Emulsionen eingesetzt werden, enthält Perlglanz gebende Mittel vorzugsweise in Mengen von 0,1 bis 7 Gew.-%, bevorzugt von 0,2 bis 6 Gew.-%, insbesondere bevorzugt 0,3 bis 5 Gew.-%, jeweils bezogen auf das Gesamtgewicht der Zusammensetzung.

Ein weiterer Gegenstand der Erfindung ist ein Verfahren zur Herstellung der kosmetischen Zusammensetzungen, bevorzugt der transparenten kosmetischen Zusammensetzungen, durch Mischen mindestens einer erfindungsgemäßen wässrigen Emulsionen mit mindestens einem Haar-Konditionierungsmittel und ggf. weiteren kosmetisch üblichen Additiven in einem kosmetisch akzeptablen Medium.

Die einzelnen Inhaltsstoffe können in einem heiß/heiß-, heiß/kalt- oder kalt/kalt-Verfahren miteinander vermischt werden.

Die Zugabe der erfindungsgemäßen wässrigen Emulsionen beim Herstellen der erfindungsgemäßen kosmetischen Zusammensetzungen erfolgt vorzugsweise bei Temperaturen von höchstens 50°C, bevorzugt bei Temperaturen von höchstens 40°C, besonders bevorzugt bei Temperaturen von höchstens 35°C. Sie erfolgt vorzugsweise bei Temperaturen von mindestens 5°C, bevorzugt bei Temperaturen von mindestens 10°C.

In den nachfolgenden Beispielen beziehen sich alle Angaben von Teilen und Prozentsätzen, soweit nicht anders angegeben, auf das Gewicht.

Sofern nicht anders angegeben, werden die folgenden Beispiele bei einem Druck der umgebenden Atmosphäre, also bei etwa 1000 hPa, und bei Raumtemperatur, also etwa 20°C bzw. einer Temperatur, die sich beim Zusammengeben der Reaktanten bei Raumtemperatur ohne zusätzliche Heizung oder Kühlung einstellt, durchgeführt.

Die Viskositäten wurden an einem Rheometer "MCR 302" der Fa. Anton Paar nach DIN EN ISO 3219: 1994 und DIN 53019 gemessen, wobei ein Kegel-Platte-System (Kegel CP50-2) mit einem Öffnungswinkel von 2° verwendet wurde. Die Kalibrierung des Gerätes erfolgte mit Normalöl 10000 der Physikalisch-Technischen Bundesanstalt. Die Messtemperatur beträgt 25,00°C +/- 0,05°C, die Messzeit 3 min. Die Viskositätsangabe stellt den arithmetischen Mittelwert von drei unabhängig durchgeführten Einzelmessungen dar. Die Messunsicherheit der dynamischen Viskosität beträgt 1,5 %. Der Schergeschwindigkeitsgradient wurde in Abhängigkeit von der Viskosität gewählt und wird für jede Viskositätsangabe separat ausgewiesen.

Die Aminzahl gibt an, wieviel mmol KOH einem Gramm der zu bestimmenden Substanz äquivalent sind. Die Bestimmung der Aminzahl erfolgt nach DIN 16945-Version 1989-03.

1H-NMR-Spektren werden als Lösung in $CDCl_3$ an einem Bruker Avance 500-NMR-Spektrometer (5 mm selektiver 1H-NMR-Probenkopf) mit einer Messfrequenz 500,13 MHz aufgenommen.

Die Auswertung erfolgt wie dem Fachmann bekannt und in folgender Literatur beschrieben: "Über die 1H-, 13C- und 29Si-NMR chemischen Verschiebungen einiger linearer, verzweigter und cyclischer Methyl-Siloxan-Verbindungen", G. Engelhardt, H. Jancke; J. Organometal. Chem. 28 (1971), 293-300; "Chapter 8 - NMR spectroscopy of organosilicon compounds", Elizabeth A. Williams, The Chemistry of Organic Silicon Compounds, 1989 John Wiley and Sons Ltd,

511-533.

Die Partikelgrößen wurden an einem Partikelgrößenmessgerät Zetasizer Nano-S, Fa. Malvern, Software Version 6.01 mittels dynamischer Lichtstreuung (Messmethode nach Mie) bestimmt. Dazu wurden die Emulsionen mit gefiltertem und entgastem Wasser auf 0,5 Gew.-% verdünnt. Die angegebenen Werte beziehen sich immer auf den Wert D(50). D(50) ist als volumengemittelter Partikeldurchmesser zu verstehen, bei dem 50% aller gemessenen Partikel einen volumengemittelten Durchmesser kleiner als der ausgewiesene Wert D(50) aufweisen.

**Beispiele:**

Beispiel 1: Carbamato-funktionalisiertes Polydimethylsiloxan **1**

1000,0 g eines reaktiv terminierten Copolymers aus Aminoethylaminopropylmethylsiloxan- und Dimethylsiloxan-Einheiten der Viskosität 4320 mPas (bei 25°C und bei einer Scherrate von 10 1/s) und der Aminzahl von 0,135 mmol/g werden in einem 2000 ml Dreihalskolben unter Stickstoffatmosphäre vorgelegt und mit 3,62 g Propylencarbonat versetzt. Die Reaktionsmischung wird 2 Stunden bei 50°C und zwei Stunden bei 60°C gerührt. Nach Abkühlen erhält man ein klares Produkt **1** der Viskosität 6200 mPas (bei 25°C und bei einer Scherrate von 10 1/s). Das 1H-NMR-Spektrum zeigt einen Funktionalisierungsgrad bzgl. Carbamat von 25 % aller zur Verfügung stehenden Aminogruppen und somit 0,058 Gew.-% Hydroxygruppen. Das Carbamato-funktionalisierte Polydimethylsiloxan **1** weist eine Aminzahl von 0,10 mmol/g auf.

Beispiel 2: Carbamato-funktionalisiertes Polydimethylsiloxan **2**

1000,0 g eines reaktiv terminierten Copolymers aus Aminoethylaminopropylmethylsiloxan- und Dimethylsiloxan-Einheiten der Viskosität 4320 mPas (bei 25°C und bei einer Scherrate von 10 1/s) und der Aminzahl von 0,135 mmol/g werden in einem 2000 ml Dreihalskolben unter Stickstoffatmosphäre vorgelegt und mit 7,24 g Propylencarbonat versetzt. Die Reaktionsmischung wird 2 Stunden bei 50°C und zwei Stunden bei 60°C gerührt. Nach Abkühlen erhält man ein klares Produkt **1** der Viskosität 9480 mPas (bei 25°C und bei einer Scherrate von 10 1/s). Das 1H-NMR-Spektrum zeigt einen Funktionalisierungsgrad bzgl. Carbamat von 47 % aller zur Verfügung stehenden Aminogruppen und somit 0,107 Gew.-% Hydroxygruppen. Das Carbamato-funktionalisierte Polydimethylsiloxan **2** weist eine Aminzahl von 0,07 mmol/g auf.

(Vergleichs-)Beispiel V3: Amino-funktionalisiertes Polydimethylsiloxan **V3**

Es handelt sich um ein reaktiv terminiertes Copolymer **V3** aus 3-(2-Aminoethylamino)propylmethylsiloxy- und Dimethylsiloxyeinheiten mit einer Aminzahl von 0,13 mmol/g und einer Viskosität von 3900 mPas (bei 25°C und bei einer Scherrate von 10 1/s).

Beispiel 4: Emulsion **E1** aus Carbamato-funktionalisiertem Polydimethylsiloxan **1**

In einem Ultra-Turrax T 50 Emulgiergerät (Fa. Janke & Kunkel/IKA) werden 12,0 g Isotridecylhexaethoxylat, käuflich erwerblich unter dem Handelsnamen Imbentin T/060 (Fa. Kolb), 7,5 g PEG-200 hydriertes Glycerylpalmat (HLB > 19; 200 Ethylenoxid-Einheiten), käuflich erwerblich unter dem Handelsnamen Rewoderm LI 520-70 (Fa. Evonik) und 8,0 g vollentsalztes Wasser vorgelegt. 30,0 g des Carbamato-funktionalisierten Produkts **1** wird in einer Portion unter einer Scherung von 4000 UmP zugegeben, so dass eine relativ feste steife Phase als Voremulsion resultiert. Es wird mit 2,3 g 80%iger Essigsäure versetzt und mit 90,93 g vollentsalztem Wasser portionsweise unter geringer Scherung zur gewünschten Emulsion verdünnt. Konservierung der Emulsion erfolgt mit 1,35 g 2-Phenoxyethanol. Es resultiert eine klare, farblose, leicht bläulich schimmernde, fließfähige Emulsion **E1** mit einer Partikelgröße D(50) von 32 nm. Die Emulsion ist auch nach 6-monatiger Lagerung bei Raumtemperatur homogen und stabil.

Beispiel 5: Emulsion **E2** aus Carbamato-funktionalisiertem Polydimethylsiloxan **2**

Carbamato-funktionalisiertes Polydimethylsiloxan **2** wird nach der gleichen Vorschrift wie in Beispiel 4 emulgiert. Es resultiert ebenfalls eine klare, farblose, leicht bläulich schimmernde, fließfähige Emulsion **E2** mit einer Partikelgröße D(50) von 30 nm. Die Emulsion ist auch nach 6-monatiger Lagerung bei Raumtemperatur homogen und stabil.

Beispiele 6-10: Emulsionen **E3** bis **E7** aus dem Carbamato-funktionalisierten Polydimethylsiloxan **2** mit unterschiedlichen Emulgatoren und Emulgator-Mengen

Die folgenden Emulsionen werden nach der gleichen Vorschrift hergestellt wie in Beispiel 4 beschrieben. Folgende Emulgatoren/Emulgator-Mengen wurden verwendet:

Beispiel 6:

12,0 g Isotridecylhexaethoxylat, käuflich erwerblich unter dem Handelsnamen Imbentin T/060 (Fa. Kolb)
3,75 g PEG-200 hydriertes Glycerylpalmat (HLB > 19; 200 Ethylenoxid-Einheiten), käuflich erwerblich unter dem Handelsnamen Rewoderm LI 520-70 (Fa. Evonik)
3,75 g Glycerin
Es resultiert eine klare, farblose, leicht bläulich schimmernde, fließfähige Emulsion E3 mit einer Partikelgröße D(50) von 21 nm.

Beispiel 7:

12,0 g Isotridecylhexaethoxylat, käuflich erwerblich unter dem Handelsnamen Imbentin T/060 (Fa. Kolb)
2,25 g PEG-200 Rizinusöl (50%ige wässrige Lösung) (HLB = 18,1; 200 Ethylenoxid-Einheiten), käuflich erwerblich unter dem Handelsnamen Cirrasol G-1300 (Fa. Croda)
Es resultiert eine klare, leicht bläuliche, gut fließfähige Emulsion **E4** mit einer Partikelgröße D(50) von 25 nm.

Beispiel 8:

12,0 g Isotridecylhexaethoxylat, käuflich erwerblich unter dem Handelsnamen Imbentin T/060 (Fa. Kolb)
7,25 g PEG-200 Rizinusöl (50%ige wässrige Lösung) (HLB = 18,1; 200 Ethylenoxid-Einheiten), käuflich erwerblich unter dem Handelsnamen Cirrasol G-1300 (Fa. Croda)
Es resultiert eine klare, bläulich schimmernde, gut fließfähige Emulsion **E5** mit einer Partikelgröße D(50) von 22 nm.

Beispiel 9:

12,0 g Isotridecylhexaethoxylat, käuflich erwerblich unter dem Handelsnamen Imbentin T/060 (Fa. Kolb)
3,45 g PEG-100 Stearat (HLB = 18,8; 100 Ethylenoxid-Einheiten), käuflich erwerblich unter dem Handelsnamen Sympatens-BS/1000G (Fa. Kolb)
Es resultiert eine klare, bläulich schimmernde, gut fließfähige Emulsion **E6** mit einer Partikelgröße D(50) von 28 nm.

Beispiel 10:

12,0 g Isotridecylhexaethoxylat, käuflich erwerblich unter dem Handelsnamen Imbentin T/060 (Fa. Kolb)
7,50 g PEG-80 Sorbitanlaurat (HLB = 19,1; 80 Ethylenoxid-Einheiten), käuflich erwerblich unter dem Handelsnamen Tween 28 (Fa. Croda)
Es resultiert eine klare, bläulich schimmernde, gut fließfähige Emulsion **E7** mit einer Partikelgröße D(50) von 22 nm.

(Vergleichs-)Beispiel 11:

Emulsion **VE1** aus Carbamato-funktionalisiertem Polydimethylsiloxan **2**

Carbamato-funktionalisiertes Polydimethylsiloxan **2** wird nach der gleichen Vorschrift wie in Beispiel 4 emulgiert. Als Emulgatoren werden aber 12,38 g Laureth-4 (HLB = 9,7; 4 Ethylenoxid-Einheiten), käuflich erwerblich unter dem Handelsnamen Sympatens ALM/040 (Fa. Kolb), und 7,13 g Laureth-23 (HLB = 16,9 aber 23 Ethylenoxid-Einheiten), käuflich erwerblich unter dem Handelsnamen Sympatens ALM/230 G (Fa. Kolb), verwendet. Es resultiert eine weißlich, trübe, fließfähige Emulsion **VE1** mit einer Partikelgröße D(50) von 112 nm.

Vergleichsbeispiel 11 zeigt, dass bei Verwendung nichtionischer, Polyethylenoxid-haltiger Emulgatoren mit einem Gehalt an Polyethylenoxid-Einheiten von kleiner 40, auch wenn der HLB-Wert größer 15 ist, keine erfindungsgemäßen Emulsionen mit einer Partikelgröße von kleiner gleich 100 nm erhalten werden können.

(Vergleichs-)Beispiel 12: Emulsion **VE2** aus Amino-funktionalisiertem Polydimethylsiloxan **V3**

Mit Hilfe eines Dissolvers Typ LDV 1 der Firma PC Laborsystem bereitet man aus 6,5 g Isotridecylpentaethoxylat, käuflich erwerblich unter dem Handelsnamen Lutensol TO 5 (Fa. BASF), 20,0 g des Amino-funktionalisierten Polydimethylsiloxans **V3**, 2,9 g Glycerin, 0,12 g 80%iger Essigsäure, 0,19 g N-Morpholinomethyltriethoxysilan und 70 g Wasser eine Emulsion mit einer durchschnittlichen Partikelgröße (D50) von 20 bis 50 nm. In diese Emulsion werden 0,13 g 2-Phenoxyethanol eingemischt.

Testmethoden zur Beurteilung der Wirkung von kosmetischen Zusammensetzungen:

Die Beurteilung des Applikationsverhaltens der kosmetischen Zusammensetzung und deren Wirkung bezüglich Kämmkraft und Weichheit erfolgten auf kaukasischem Haar. Alle Haartressen werden vor ihrem Einsatz gereinigt. Hierzu werden die Haartressen 24 Stunden in ein Lösungsmittelgemisch aus gleichen Teilen Aceton, Ethanol, Isopropanol und voll entsalztem Wasser eingelegt. Nach Entfernen des Lösungsmittelgemischs werden die Haartressen gründlich mit voll entsalztem Wasser gewaschen. Anschließend wird jede Haartresse mit 3 ml einer Ammonium Lauryl Sulfat Lösung (25%ig), STEPANOL(R) ALS 25, Fa. STEPAN Company, gewaschen und so lange mit voll entsalztem Wasser gespült, bis kein Schaum mehr sichtbar ist (mindestens 2 Minuten). Nach der Grundreinigung werden die Tressen vor ihrer Weiterverwendung mindestens 12 Stunden bei 23°C und 60% Luftfeuchte konditioniert.

Kämmkraftmessung:

Zur Bestimmung der Kämmkraft von nassem und trockenem Haar wurden Haartressen aus geschädigtem, kaukasischem Haar der Fa. Kerling International Haarfabrik GmbH (Haartressen Schädigungsgrad B, doppelt gezogen) mit einem Gewicht von 2 g und einer Länge von 20 cm verwendet. Die Messung der Kämmkraft mit Doppelkammmethode nach Y. K. Kamath und Hans-Dietrich Weigmann, J. Soc. Cosmet. Chem., 37, 111-124, 1986 erfolgte mit einer Zug-Dehnungsmaschine Instron 3343. Zunächst wird die Nass- und Trockenkämmkraft entlang der Messtrecke an unbehandelten Haartressen bestimmt. Anschließend werden die Haartressen mit einer erfindungsgemäßen kosmetischen Zusammensetzung behandelt und die Kraftaufnahme beim Kämmvorgang bestimmt. Als Messwert wird die Reduktion der Kämmkraft entlang der Messtrecke (Arbeit) angegeben, die sich zwischen der behandelten und unbehandelten Haartresse ergibt. Es wird der Mittelwert aus drei Haartressen gebildet. Die Angabe der Kämmkraftreduktion erfolgt in Prozent.

Geschmeidigkeit:

Das Messprinzip zur Bestimmung der Hydrophobie von Haaren folgt den Ausführungen in DE 10 2010 020 192. Zur Bestimmung der Weichheit des Haars wurden Haartressen aus geschädigtem, kaukasischem Haar der Fa. Kerling International Haarfabrik GmbH (Haartressen Schädigungsgrad B, doppelt gezogen) mit einem Gewicht von 2 g und einer Länge von 20 cm verwendet. Die Bestimmung der Haarweichheit im trockenen Zustand erfolgte durch Einsatz einer Zug-Prüfmaschine Instron 3343, indem die benötigte Zugkraft mit den Parametern Biegesteifigkeit und Oberflächenrauheit des Haarbündels in Korrelation gesetzt wird. Diese beiden Parameter wiederum korrelieren mit der Haarweichheit. Zu diesem Zweck wurde eine unbehandelte Haartresse in eine Messanordnung eingespannt, die aus fünf versetzt gegenüberliegenden Stäben besteht. Die Form der Haartresse in dieser Ausgangsposition ist eine Art Doppel-S. Die Haartresse wird nach dieser Vorbereitung in einer Richtung aus der Messanordnung gezogen und die notwendige Kraft entlang der Messtrecke als Arbeit ausgewertet. Anschließend werden die Haartressen mit einer erfindungsgemäßen kosmetischen Zusammensetzung behandelt und die Kraftaufnahme beim Ziehen der Haartresse durch die Messanordnung entlang der Messstrecke bestimmt. Als Messwert wird die Reduktion der Zugkraft entlang der Messtrecke (Arbeit) angegeben, die sich zwischen der behandelten und unbehandelten Haartresse ergibt. Eine hohe Reduktion der Zugkraft (Arbeit) entspricht einem guten Weichgriff bzw. einer hohen Geschmeidigkeit. Es wird der Mittelwert aus drei Haartressen gebildet.

Aufwaschprozedur Shampoo:

Auf eine gereinigte, befeuchtete Haartresse wird 0,1 g Shampoo pro g Haar appliziert. Das Shampoo wird 30 Sekunden lang in Richtung der Haarspitzen einmassiert. Anschließend wird die Haartresse 30 s unter fließendem, voll entsalztem Wasser ausgespült und mit einem grobzinkigen Kamm entwirrt. Die Prozedur wird zweimal wiederholt. Beim letzten Mal wird der Spülprozess auf 60 s verlängert. Anschließend wird die Haartresse mindestens 12 h bei einer Luftfeuchte von 60% und einer Temperatur von 23°C getrocknet.

Im Folgenden beziehen sich alle Angaben in Teilen auf Gew.-teile.

Beispiel 13A bis 13B:

Kosmetische Zusammensetzung: Shampoo

Die nachfolgenden Beispiele repräsentieren kosmetische Zusammensetzungen enthaltend Emulsionen **E1** bis **E2** aus Beispiel 4 bis Beispiel 5. Der Aktivgehalt an Organopolysiloxan in der kosmetischen Zusammensetzung beträgt 0,66 %.

Tabelle 1: Kosmetische Zusammensetzungen (Shampoo) **F1** und **F2**

| Bestandteile (INCI-Name) | Beispiel 13 A kosmetische Zusammensetzung **F1** [Gew.-teile] | Beispiel 13 b kosmetische Zusammensetzung **F2** [Gew.-teile] |
|---|---|---|
| Aqua (DI Water) | ad 100 | ad 100 |
| Guar Hydroxypropyltrimonium Chloride 1) | 0,2 | 0,2 |
| Lauryl Polyglucose, Lauryl Glucoside 2) | 21,4 | 21,4 |
| Glycol Distearate 3) | 1,2 | 1,2 |
| PEG-150 Distearate 4) | 0,2 | 0,2 |
| Citric Acid 5) | 0,2 | 0,2 |
| Cocamidopropyl Betaine 6) | 13,4 | 13,4 |
| Emulsion **E1** aus Beispiel 4 | 3,3 | |
| Emulsion **E2** aus Beispiel 5 | | 3,3 |
| Methylchloroisothiazolinone, Methylisothiazolinone 7) | 0,1 | 0,1 |

1) Guar Hydroxypropyltrimonium Chloride: N-Hance(R) 3000, Hercules Inc.
2) Lauryl Polyglucose, Lauryl Glucoside: Plantacare(R) 1200 UP; BASF SE
3) Glycol Distearate: Genapol(R) PMS, Clariant GmbH
4) PEG-150 Distearate: Eumulgin(R) EO 33, BASF SE
5) Citric Acid: Citric Acid, Sigma
6) Cocamidopropyl Betaine: Amphosol CG, STEPAN Company
7) Methylchloroisothiazolinone, Methylisothiazolinone: Kathon™ CG, Rohm and Haas Company, Inc.

Herstellanweisung:

Guar Hydroxypropyltrimonium Chlorid wird in Wasser dispergiert und die Mischung wird auf 75 °C erwärmt. Anschließend wird Lauryl Polyglucose, Lauryl Glucoside, Glycol Distearate und PEG-150 Distearate zugegeben. Nach fünfminütigem Rühren bei 75°C wird die Lösung unter langsamem Rühren auf 35 °C abgekühlt. Nach Erreichen dieser Zieltemperatur wird Emulsion **E1** (analoges Vorgehen bei Emulsion **E2**), Zitronensäure, Cocamidopropyl Betain und der Konservierer Methylchloroisothiazolinone, Methylisothiazolinone zugegeben und auf Raumtemperatur abgekühlt.

Als Vergleichszusammensetzung **VF3** wird die kosmetische Zusammensetzung aus Tabelle 1 verwendet. Statt der Emulsionen **E1** bis **E2** werden 5,0 Teile der Emulsion **VE2** einformuliert.

Die Viskositäten der erhaltenen kosmetischen Zusammensetzungen **F1** bis **F2** sowie **VF3** werden sofort nach Herstellung bzw. nach 12 Tagen bestimmt.

Wie folgende Tabelle 2 zeigt, führt der Einsatz der erfindungsgemäßen Emulsionen **E1** und **E2** in den kosmetischen Zusammensetzungen **F1** und **F2** zu keinem oder nur leichten Absinken der Viskositäten der Zusammensetzungen im Laufe der Zeit. Dagegen ist bei der Vergleichszusammensetzung **VF3**, in der anstelle eines Carbamato-funktionalisierten Polydimethylsiloxans ein Amino-funktionalisiertes Polydimethylsiloxan eingesetzt wurde, ein deutlicheres Absinken der Viskosität zu erkennen. Die kosmetischen Zusammensetzungen **F1** und **F2**, die die erfindungsgemäßen Emulsionen **E1** und **E2** enthalten, weisen somit eine höhere Stabilität auf.

Tabelle 2: Bestimmung der Viskosität der kosmetischen Zusammensetzungen

| Kosmetische Zusammensetzung | Viskosität [mPas] direkt nach Herstellung | Viskosität [mPas] nach 12 Tagen | Absinken der Viskosität [mPas] |
|---|---|---|---|
| **F1** | 1550 | 1540 | - 10 |
| **F2** | 1680 | 1512 | - 168 |
| **VF3** | 2771 | 2280 | - 491 |

Beispiel 14:

Kosmetische Zusammensetzung: Shampoo

Das nachfolgende Beispiel repräsentiert eine kosmetische Zusammensetzung **F4** enthaltend Emulsion **E2** aus Beispiel 5. Der Aktivgehalt an Organopolysiloxan in der kosmetischen Zusammensetzung beträgt 2 %.

Tabelle 3: Kosmetische Zusammensetzung (Shampoo) **F4**

| Bestandteile (INCI-Name) | kosmetische Zusammensetzung (Shampoo) **F4** [Gew.-teile] |
|---|---|
| Aqua (DI Water) | Ad 100 |
| Guar Hydroxypropyltrimonium Chlorid 1) | 0,2 |
| Sodium Laureth Sulfat 2) | 37,74 |
| Cocamide MIPA 3) | 0,5 |
| Glycol Distearat 4) | 1,40 |
| PEG-150 Distearat 5) | 0,20 |
| Cocamidopropyl Betaine 6) | 10,07 |
| Emulsion **E2** aus Beispiel 5 | 10 |
| Methylchloroisothiazolinone, Methylisothiazolinone 7) | 0,06 |
| Natriumchlorid | 0,15 |

1) Guar Hydroxypropyltrimonium Chloride: N-Hance® 3000, Hercules Inc.
2) Sodium Laureth Sulfat: Genapol® LRO 26,5%, Clariant
3) Cocamide MIPA: NINOL M-10, STEPAN Company
4) Glycol Distearate: Genapol® PMS, Clariant GmbH
5) PEG-150 Distearate: Eumulgin® EO 33, BASF SE
6) Cocamidopropyl Betaine: Amphosol CG, STEPAN Company
7) Methylchloroisothiazolinone, Methylisothiazolinone: Kathon™ CG, Rohm and Haas Company, Inc.

Herstellanweisung:

Guar Hydroxypropyltrimonium Chlorid wird in Wasser dispergiert und die Mischung wird auf 75 °C erwärmt. Anschließend wird Natrium Laureth Sulfat, Cocamide MIPA, Glycol Distearat und PEG-150 Distearate zugegeben. Nach fünfminütigem Rühren bei 75°C wird die Lösung unter langsamem Rühren auf 35 °C abgekühlt. Nach Erreichen dieser Zieltemperatur wird Emulsion E2, Cocamidopropyl Betain und der Konservierer Methylchloroisothiazolinone, Methylisothiazolinone zugegeben und auf Raumtemperatur abgekühlt. Abschließend erfolgt die Zugabe von Natriumchlorid unter Rühren.

Vergleichsbeispiel:

Als Vergleichszusammensetzung **VF5** wird die kosmetische Zusammensetzung aus Tabelle 3 ohne Emulsion **E2** verwendet, also die siliconfreie Shampoobasis.

Tabelle 4 enthält die Ergebnisse der Messung für die Parameter Trockenkämmkraft, Nasskämmkraft und Geschmeidigkeit an Haaren behandelt mit der erfindungsgemäßen kosmetischen Zusammensetzung **F4** sowie mit der

nicht-erfindungsgemäßen kosmetischen Zusammensetzung **VF5** im Vergleich zu unbehandelten Haaren.

Tabelle 4: Messung der Trockenkämmkraft, Nasskämmkraft und Geschmeidigkeit

| Kosmetische Zusammensetzung | Verringerung der Trockenkämmkraft (Arbeit) im Vergleich zu unbehandeltem Haar in % | Verringerung der Nasskämmkraft (Arbeit) im Vergleich zu unbehandeltem Haar in % | Erhöhung der Geschmeidigkeit (Arbeit) im Vergleich zu unbehandeltem Haar in % |
|---|---|---|---|
| **F4** | 65 | 60 | 27 |
| **VF5** | 33 | 32 | -3 |

Tabelle 4 zeigt, dass der Einsatz der erfindungsgemäßen Emulsion **E2** in den kosmetischen Zusammensetzungen **F4** zu einer deutlichen Verbesserung der Pflegeeigenschaften führt. Der Einsatz der erfindungsgemäßen Emulsionen **E2** bewirkt eine Reduzierung der aufzuwendenden Kämmkräfte im trockenen und nassen Haar. Die Verringerung der Trocken- und Nasskämmkraft bei der Behandlung mit der erfindungsgemäßen Zusammensetzung **F4** ist deutlich größer im Vergleich zur Behandlung mit der nicht-erfindungsgemäßen Zusammensetzung **VF5.** Weiterhin wird die Geschmeidigkeit des Haars bei der Behandlung mit der erfindungsgemäßen Zusammensetzung **F4** im Vergleich zu einer Behandlung mit der nicht-erfindungsgemäßen kosmetischen Zusammensetzung **VF5,** die keine erfindungsgemäße Emulsion enthält, verbessert.

Beispiele 15A und 15B:

Kosmetische Zusammensetzung: Transparente Shampoos

Die nachfolgenden Beispiele repräsentieren kosmetische Zusammensetzungen enthaltend Emulsion **E1** aus Beispiel 4 und Emulsion **E2** aus Beispiel 5. Der Aktivgehalt an Organopolysiloxan in der kosmetischen Zusammensetzung beträgt 0,5 %.

Tabelle 5: Kosmetische Zusammensetzungen (transparente Shampoos) **F6** und **F7**

| Bestandteile (INCI-Name) | Beispiel 15A kosmetische Zusammensetzung **F6** [Gew.-teile] | Beispiel 15B kosmetische Zusammensetzung **F7** [Gew.-teile] |
|---|---|---|
| Aqua (DI Water) | Ad 100 | Ad 100 |
| Polyquaternium-10 1) | 0,10 | 0,10 |
| Cocamide MEA | 1,00 | 1,00 |
| Sodium Laureth Sulfate 3) | 52,80 | 52,80 |
| Cocamidopropyl Betain 4) | 10,06 | 10,06 |
| Methylchloroisothiazolinone, Methylisothiazolinone 5) | 0,06 | 0,06 |
| Emulsion **E1** aus Beispiel 4 | 2,50 | |
| Emulsion **E2** aus Beispiel 5 | | 2,50 |
| Aqua (DI Water) | 15,00 | 15,00 |
| Sodium Chloride | 1,00 | 1,00 |

1) Polyquaternium-10: UcareTM Polymer JR-400, Amerchol Corporation
2) Cocamide MEA: COMPERLAN® 100, Cognis Coorporation
3) Sodium Laureth Sulfat: Genapol® LRO 26,5%, Clariant
4) Cocamidopropyl Betaine: Amphosol CG, STEPAN Company
5) Methylchloroisothiazolinone, Methylisothiazolinone: Kathon™ CG, Rohm and Haas Company, Inc.

Herstellanweisung:

Polyquaternium-10 wird in Wasser dispergiert und die Mischung wird unter Rühren auf 70 °C erwärmt. Anschließend wird Natrium Laureth Sulfat und Cocamide MEA zugegeben. Nach dem Abkühlen der Mischung unter langsamem Rühren auf 35 °C wird Cocamidopropylbetain und der Konservierer Methylchloroisothiazolinone, Methylisothiazolinone zugegeben. Im Anschluss wird die in Wasser vorverdünnte Emulsion **E1** bzw. Emulsion **E2** zugegeben. Durch Zugabe von Natriumchlorid wird die Viskosität des Shampoos auf den Zielbereich von ca. 10000 mPas eingestellt.

Nach Behandlung von geschädigten kaukasischen Haaren mit den Shampoos **F6** und **F7** aus Beispielen 15A und 15B fühlen sich diese weicher an als unbehandeltes Haar.

Beispiele 16A - 16G:

Kosmetische Zusammensetzung: Rinse-Off Conditioner 16A - 16G Das nachfolgende Beispiel repräsentiert kosmetische Zusammensetzungen **F8** - **F14** gemäß Tabelle 6 enthaltend die Emulsionen **E1** - **E7** aus den Beispielen 4 bis 10. Der Aktivgehalt an Organopolysiloxan in der kosmetischen Zusammensetzung beträgt 0,5 bis 2 %.

Tabelle 6: Kosmetische Zusammensetzung: Rinse-Off Conditioner **F8** - **F14**

| **Bestandteile (INCI-name)** | Bsp. 16A Kosmet. Formul. **F8** [Gew.-teile] | Bsp. 16B Kosmet. Formul. **F9** [Gew.-teile] | Bsp. 16C Kosmet. Formul. **F10** [Gew.-teile] | Bsp. 16D Kosmet. Formul. **F11** [Gew.-teile] | Bsp. 16E Kosmet. Formul. **F12** [Gew.-teile] | Bsp. 16F Kosmet. Formul. **F13** [Gew.-teile] | Bsp. 16G Kosmet. Formul. **F14** [Gew.-teile] |
|---|---|---|---|---|---|---|---|
| Water | ad 100 | ad 100 | ad 100 | ad 100 | ad 100 | ad 100 | ad 100 |
| Hydroxyethyl-cellulose 1) | 1,2 | 1,2 | 1,2 | 1,2 | 1,2 | 1,2 | 1,2 |
| Cetyl Alcohol 2) | 1,0 | 1,0 | 1,0 | 1,0 | 1,0 | 1,0 | 1,0 |
| Polysorbate 80 3) | 1,0 | 1,0 | 1,0 | 1,0 | 1,0 | 1,0 | 1,0 |
| Behentrimonium Chloride 4) | 1,8 | 1,8 | 1,8 | 1,8 | 1,8 | 1,8 | 1,8 |
| Stearamidopropyl Dimethylamine 5) | 0,5 | 0,5 | 0,5 | 0,5 | 0,5 | 0,5 | 0,5 |
| Stearyl Alcohol 6) | 3,0 | 3,0 | 3,0 | 3,0 | 3,0 | 3,0 | 3, 0 |
| Citric Acid 7) | 0,2 | 0,2 | 0,2 | 0,2 | 0,2 | 0,2 | 0,2 |
| Tetrasodium EDTA 8) | 0,2 | 0,2 | 0,2 | 0,2 | 0,2 | 0,2 | 0,2 |
| Emulsion **E1** aus Bsp. 4 | 10,0 | | | | | | |
| Emulsion **E2** aus Bsp. 5 | | 5, 0 | | | | | |
| Emulsion **E3** aus Bsp. 6 | | | 7,5 | | | | |
| Emulsion **E4** aus Bsp. 7 | | | | 10,0 | | | |
| Emulsion **E5** aus Bsp. 8 | | | | | 2,5 | | |
| Emulsion **E6** aus Bsp. 9 | | | | | | 5,0 | |
| Emulsion **E7** aus Bsp. 10 | | | | | | | 5, 0 |

(fortgesetzt)

| **Bestandteile (INCI-name)** | Bsp. 16A Kosmet. Formul. **F8** [Gew.-teile] | Bsp. 16B Kosmet. Formul. **F9** [Gew.-teile] | Bsp. 16C Kosmet. Formul. **F10** [Gew.-teile] | Bsp. 16D Kosmet. Formul. **F11** [Gew.-teile] | Bsp. 16E Kosmet. Formul. **F12** [Gew.-teile] | Bsp. 16F Kosmet. Formul. **F13** [Gew.-teile] | Bsp. 16G Kosmet. Formul. **F14** [Gew.-teile] |
|---|---|---|---|---|---|---|---|
| Phenoxyethanol, Ethylhexylglycerin 9) | 0,9 | 0,9 | 0,9 | 0,9 | 0,9 | 0,9 | 0,9 |

Die in Tabelle 6 genannten Rohstoffe sind unter folgenden Handelsnamen erhältlich:

1)Hydroxyethylcellulose: Tylose® H 4000 P2, Shin-Etsu Chem. Co.
2)Cetylalkohol: Cetylalkohol, Merck KGaA
3)Polysorbate 80: Tween™ 80, Croda GmbH
4)Behentrimonium Chloride: Genamin® KDMP, Clariant GmbH
5)Stearamidopropyl Dimethylamine, Incromine™ SB , Croda GmbH
6)Stearylalkohol: Stearylalkohol Merck KGaA
7)Citric Acid: Citric Acid, Sigma
8)Tetrasodium EDTA: EDETA® B Pulver, BASF Corporation
9)Phenoxyethanol, Ethylhexylglycerin: Euxyl PE 9010, Schülke & Mayr

Herstellanweisung:

Wasser wird vorgelegt und unter Rühren auf 75°C erwärmt. Dabei werden 1,2 Teile Hydroxyethylcellulose zugegeben. Wenn 65°C erreicht sind, werden 0,5 Teile Stearamidopropyl Dimethylamine, 1 Teil Polysorbate 80, 3 Teile Stearyl Alcohol, 1 Teil Cetyl Alcohol und 1,8 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,2 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion aus den Beispielen. Die Zusammensetzung wird 15 Minuten unter Rühren homogenisiert.

Nach Behandlung von geschädigten kaukasischen Haaren mit den Rinse-Off Conditionern **F8** bis **F14** aus Beispielen 16A bis 16G fühlen sich diese weicher an als unbehandeltes Haar.

## Patentansprüche

1. Wässrige Emulsionen enthaltend

(A) Carbamato-funktionalisierte Organopolysiloxane, die durchschnittlich je Molekül mindestens eine Carbamatofunktionelle Gruppe Y der Formel

$$-R^4-[NX-R^5-]_nNX^-H$$

enthalten, wobei
X gleich oder verschieden ist und ein Wasserstoffatom oder einen Rest Z der Formel

$$-CO-O-CHR^6-CH_2-OH \text{ oder } -CO-O-CH_2-CHR^6-OH$$

bedeutet, wobei durchschnittlich je Molekül mindestens ein Rest X ein Rest Z ist,
wobei $R^4$ gleich oder verschieden ist und einen zweiwertigen, Si-C-gebundenen Kohlenwasserstoffrest mit 1 bis 18 C-Atomen bedeutet,
$R^5$ gleich oder unterschiedlich ist und einen zweiwertigen Kohlenwasserstoffrest mit 1 bis 6 C-Atomen bedeutet,
$R^6$ gleich oder unterschiedlich ist und ein Wasserstoffatom oder einen einwertigen Kohlenwasserstoffrest mit 1 bis 36 C-Atomen bedeutet und
n gleich 1, 2, 3 oder 4, vorzugsweise 1, ist,
mit der Maßgabe, dass bei den Carbamato-funktionalisierten Organopolysiloxanen (A) weniger als 50 Mol% der N-gebundenen Reste X in den Gruppen Y nicht ein Wasserstoffatom sind, sondern die obige Bedeutung des Restes Z haben,

(B) nicht-ionische, Polyethylenoxid-haltige Emulgatoren, die mehr als 40 Ethylenoxid-Einheiten der Formel $-CH_2-CH_2-O-$ enthalten und einen HLB-Wert von größer oder gleich 15 aufweisen, ausgewählt aus der Gruppe von Alkylpolyglycolether, Carbonsäurepolyglycolester,
Ethoxylierte Sorbitanfettsäureester,
Ethoxyliertes Rizinusöl oder hydrierte Varianten, Ethoxylierte Fettamine,
Ethoxylierte Glyceryl Fettsäurecarboxylate, Blockcopolymere aus Ethylenoxid- und Propylenoxid-Einheiten, die

als Poloxamere bezeichnet werden, und Copolymere aus Ethylenoxid- und Propylenoxid-Einheiten, die über einen Ethylendiamin-Kern verbrückt sind und als Poloxamine bezeichnet werden,
und
(C) Wasser,
wobei die Emulsionen Partikelgrößen von kleiner oder gleich 100 nm (D50) gemessen gemäß Beschreibung aufweisen.

2. Wässrige Emulsionen nach Anspruch 1, **dadurch gekennzeichnet, dass** als Carbamato-funktionalisierte Organopolysiloxane (A) Carbamato-funktionalisierte Polydiorganosiloxane der Formel

$$[R^1_2R^2SiO_{1/2}]_2[R^2(Y)SiO_{2/2}]_k[R^1_2SiO_{2/2}]_m \quad (I),$$

wobei

$R^1$ gleich oder verschieden ist und einen einwertigen Si-C-gebundenen Kohlenwasserstoffrest mit 1 bis 18 C-Atomen bedeutet,
$R^2$ gleich oder unterschiedlich ist und gleich Rest $R^1$ oder eine Hydroxygruppe -OH oder eine Alkoxy-Gruppe der Formel-O-$R^3$ bedeutet, wobei $R^3$ ein gegebenenfalls substituierter Alkylrest mit 1-8 C-Atomen ist,
Y die im Anspruch 1 dafür angegebene Bedeutung hat, wobei durchschnittlich je Molekül mindestens eine Gruppe Y einen Rest Z der Formel

$$-CO-O-CHR^6-CH_2-OH \text{ oder } -CO-O-CH_2-CHR^6-OH$$

enthält, wobei $R^6$ die im Anspruch 1 dafür angegebene Bedeutung hat,
m eine ganze Zahl und mindestens 40 und höchstens 1000 ist
k eine ganze Zahl und mindestens 1 und höchstens 15 ist,
wobei das Verhältnis von m zu k mindestens 65 und höchstens 1000 ist,
eingesetzt werden.

3. Wässrige Emulsionen nach Anspruch 1 oder 2, **dadurch gekennzeichnet, dass** $R^6$ ein Methylrest ist.

4. Wässrige Emulsionen nach Anspruch 1, 2 oder 3, **dadurch gekennzeichnet, dass** bei den Carbamato-funktionalisierten Organopolysiloxanen (A) mindestens 5 Mol% der N-gebundenen Reste X in den Gruppen Y nicht ein Wasserstoffatom sind, sondern die Bedeutung des Restes Z der Formel

$$-CO-O-CHR^6-CH_2-OH \text{ oder } -CO-O-CH_2-CHR^6-OH$$

haben, wobei $R^6$ die im Anspruch 1 dafür angegebene Bedeutung hat.

5. Wässrige Emulsionen nach einem der Ansprüche 1 bis 4, **dadurch gekennzeichnet, dass** die Carbamato-funktionalisierten Organopolysiloxane (A) höchstens 0,7 Gew.-%, vorzugsweise höchstens 0,32 Gew.-%, bevorzugt höchstens 0,17 Gew.-%, Hydroxygruppen aufweisen.

6. Wässrige Emulsionen nach einem der Ansprüche 1 bis 5, **dadurch gekennzeichnet, dass** die Carbamato-funktionalisierten Organopolysiloxane (A) eine Aminzahl von höchstens 0,3 mmol/g, besonders bevorzugt höchstens 0,10 mmol/g aufweisen.

7. Wässrige Emulsionen nach einem der Ansprüche 1 bis 6, **dadurch gekennzeichnet, dass** als nicht-ionische, Polyethylenoxid-haltige Emulgatoren (B), die mehr als 40 Ethylenoxid-Einheiten der Formel $-CH_2-CH_2-O-$ enthalten und einen HLB-Wert von größer oder gleich 15 aufweisen, solche ausgewählt aus der Gruppe von
Alkylpolyglycolether mit Alkylresten von 8 bis 20 C-Atomen, bevorzugt Steareth-100 (9005-00-9), Talloweth-50, Talloweth-80 (61791-28-4), Trideceth-50 (24938-91-8), als Carbonsäurepolyglycolester Fettsäurepolyglycolester, bevorzugt solche mit Carbonsäureresten von 8 bis 20 C-Atomen, insbesondere PEG-75 Oleat, PEG-200 Oleat, PEG-300 Monooleat, PEG-400 Oleat, PEG-150 Laurat, PEG-400 Laurat, PEG-75 Stearat, PEG-100 Stearat, PEG-600 Stearat, PEG-150 Distearat,
als Ethoxylierte Sorbitanfettsäureester PEG-40 Sorbitanoleat, PEG-80 Sorbitanlaurat,
als Ethoxyliertes Rizinusöl oder hydrierte Varianten PEG 75 Castor Oil oder PEG200 Castor Oil bzw. PEG-80

hydrogenated Castor Oil, PEG-100 hydrogenated Castor Oil, PEG-200 hydrogenated Castor Oil,
als Ethoxylierte Fettamine PEG-100 Talg-alkylamin (61791-44-4), PEG-40 Stearylamin, und
als Ethoxylierte Glyceryl Fettsäurecarboxylate PEG-40 Glyceryllaurat, PEG-200 Glycerylstearat, PEG-200 Glyceryltallowat und PEG-200 hydriertes Glycerylpalmat, eingesetzt werden.

**8.** Wässrige Emulsionen nach einem der Ansprüche 1 bis 7, **dadurch gekennzeichnet, dass** die Carbamato-funktionalisierten Organopolysiloxane (A) hergestellt werden durch Umsetzung von Amino-funktionalisierten Organopolysiloxanen (A'), die durchschnittlich je Molekül mindestens eine Gruppe Y' der Formel

$$-R^4-[NH-R^5-]_nNH_2$$

enthalten, wobei $R^4$, $R^5$ und n die in Anspruch 1 dafür angegebene Bedeutung haben, mit cyclischen Carbonaten der Formel

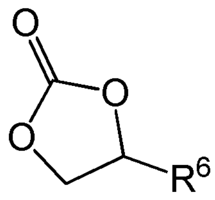

wobei
$R^6$ die in Anspruch 1 oder 3 dafür angegebene Bedeutung hat.

**9.** Wässrige Emulsionen nach Anspruch 8, **dadurch gekennzeichnet, dass** als Amino-funktionalisierte Organopolysiloxane (A') Amino-funktionalisierte Polydiorganosiloxane (A') der Formel

$$[R^1{}_2R^2SiO_{1/2}]_2[R^2(Y')SiO_{2/2}]_k[R_2SiO_{2/2}]_m \qquad (I'),$$

wobei

$R^1$, $R^2$, m und k die im Anspruch 2 dafür angegebene Bedeutung haben und
Y' die im Anspruch 8 dafür angegebene Bedeutung hat, eingesetzt werden.

**10.** Wässrige Emulsionen nach Anspruch 8 oder 9, **dadurch gekennzeichnet, dass** als Amino-funktionalisierte Organopolysiloxane (A') solche eingesetzt werden, die eine Aminzahl von höchstens 0,4 mmol/g, insbesondere höchstens 0,2 mmol/g, aufweisen.

**11.** Kosmetische Zusammensetzungen enthaltend,
in einem kosmetisch akzeptablen Medium, vorzugsweise Wasser,
mindestens ein Haar-Konditionierungsmittel und
mindestens eine wässrige Emulsion nach einem der Ansprüche 1 bis 7 oder hergestellt nach einem der Ansprüchen 8 bis 10.

**12.** Kosmetische Zusammensetzungen, nach Anspruch 11, **dadurch gekennzeichnet, dass** als Haar-Konditionierungsmittel solche ausgewählt aus der Gruppe von
kationischen Polymeren,
kationischen Tensiden,
nicht polymer vorliegenden quaternären Ammoniumverbindungen,
Organopolysiloxanen und Organopolysiloxancopolymeren, die von den in den wässrigen Emulsionen vorliegenden Carbamato-funktionalisierten Polydiorganosiloxanen (A) verschieden sind,
Fettsäureestern und Fettsäurealkoholen,
natürlichen oder synthetischen Ölen und Wachsen und Panthenol, Lipiden, Proteinen und hydrolysierten Proteinen, sowie deren Mischungen
eingesetzt werden.

**13.** Kosmetische Zusammensetzungen, nach Anspruch 11 oder 12,

**dadurch gekennzeichnet, dass** sie weitere kosmetisch übliche Additive, ausgewählt aus der Gruppe von
Tensiden, wie anionische, nichtionische und amphotere Tenside,
Verdickern, Gelierungsmitteln,
Filmbildner,
feuchtigkeitsspendenden Mitteln,
UV-Filter,
perlglanzgebenden Mitteln,
Vitaminen,
Antioxidantien,
Coffein,
Anti-Schuppenwirkstoffen,
Konservierungsmitteln
und deren Mischungen
enthalten.

**14.** Verfahren zur Herstellung der kosmetischen Zusammensetzung durch Mischen mindestens einer wässrigen Emulsion von Carbamato-funktionalisierten Organopolysiloxanen nach einem der Ansprüche 1 bis 7 oder hergestellt nach einem der Ansprüche 8 bis 10
mit mindestens einem Konditionierungsmittel und ggf. weiteren kosmetisch üblichen Additiven in einem kosmetisch akzeptablen Medium, vorzugsweise Wasser.

**15.** Verfahren zur Behandlung von keratinischen Fasern, bevorzugt Haaren, mit kosmetischen Zusammensetzungen nach einem der Ansprüche 11 bis 13 oder hergestellt nach Anspruch 14.

## Claims

**1.** Aqueous emulsions comprising

(A) carbamato-functionalized organopolysiloxanes containing an average per molecule of at least one carbamato-functional Y group of the formula

$$-R^4-[NX-R^5-]_nNX^-H$$

where
X is the same or different and is a hydrogen atom or is a Z radical of the formula

$$-CO-O-CHR^6-CH_2-OH$$

or

$$-CO-O-CH_2-CHR^6-OH$$

where an average of at least one X radical per molecule is a Z radical,
where $R^4$ is the same or different and is a divalent, Si-C-bonded hydrocarbyl radical having 1 to 18 carbon atoms,
$R^5$ is the same or different and is a divalent hydrocarbyl radical having 1 to 6 carbon atoms, $R^6$ is the same or different and is a hydrogen atom or a monovalent hydrocarbyl radical having 1 to 36 carbon atoms, and n is 1, 2, 3 or 4, preferably 1,
with the proviso that, in the carbamato-functionalized organopolysiloxanes (A), less than 50 mol% of the N-bonded X radicals in the Y groups are not a hydrogen atom, but have the above meaning of the Z radical,

(B) nonionic, polyethylene oxide-containing emulsifiers containing more than 40 ethylene oxide units of the formula $-CH_2-CH_2-O-$, and having an HLB value of not less than 15, selected from the group of
alkyl polyglycol ethers,
carboxylic acid polyglycol esters, ethoxylated sorbitan fatty acid esters, ethoxylated castor oil or hydrogenated variants, ethoxylated fatty amines, ethoxylated glyceryl fatty acid carboxylates, block copolymers of ethylene

oxide and propylene oxide units that are referred to as poloxamers, and
copolymers of ethylene oxide and propylene oxide units bridged via an ethylenediamine core that are referred to as poloxamines,
and
(C) water,
where the emulsions have particle sizes of not more than 100 nm (D50) measured according to the description.

2. Aqueous emulsions according to Claim 1, **characterized in that** the carbamato-functionalized organopolysiloxanes (A) used are carbamato-functionalized polydiorganosiloxanes of the formula

$$[R^1{}_2R^2SiO_{1/2}]_2[R^2(Y)SiO_{2/2}]_k[R^1{}_2SiO_{2/2}]_m \qquad (I)$$

where

$R^1$ is the same or different and is a monovalent Si-C-bonded hydrocarbyl radical having 1 to 18 carbon atoms,
$R^2$ is the same or different and is an $R^1$ radical or a hydroxyl group -OH or alkoxy group of the formula $-O-R^3$ where $R^3$ is an optionally substituted alkyl radical having 1-8 carbon atoms,
Y is as defined in Claim 1,
where an average of at least one Y group per molecule contains a Z radical of the formula

$$-CO-O-CHR^6-CH_2-OH$$

or

$$-CO-O-CH_2-CHR^6-OH$$

where $R^6$ is as defined in Claim 1,
m is an integer and is at least 40 and at most 1000,
k is an integer and is at least 1 and at most 15, where the ratio of m to k is at least 65 and at most 1000.

3. Aqueous emulsions according to Claim 1 or 2, **characterized in that** $R^6$ is a methyl radical.

4. Aqueous emulsions according to Claim 1, 2 or 3, **characterized in that**, in the carbamato-functionalized organopolysiloxanes (A), at least 5 mol% of the N-bonded X radicals in the Y groups are not a hydrogen atom, but have the meaning of the Z radical of the formula

$$-CO-O-CHR^6-CH_2-OH$$

or

$$-CO-O-CH_2-CHR^6-OH$$

where $R^6$ is as defined in Claim 1.

5. Aqueous emulsions according to any of Claims 1 to 4, **characterized in that** the carbamato-functionalized organopolysiloxanes (A) have at most 0.7% by weight, preferably at most 0.32% by weight, more preferably at most 0.17% by weight, of hydroxyl groups.

6. Aqueous emulsions according to any of Claims 1 to 5, **characterized in that** the carbamato-functionalized organopolysiloxanes (A) have an amine value of at most 0.3 mmol/g, more preferably at most 0.10 mmol/g.

7. Aqueous emulsions according to any of Claims 1 to 6, **characterized in that** the nonionic, polyethylene oxide-containing emulsifiers (B) used which contain more than 40 ethylene oxide units of the formula $-CH_2-CH_2-O-$ and have an HLB value of not less than 15 are those selected from the group of alkyl polyglycol ethers having alkyl radicals of 8 to 20 carbon atoms, preferably Steareth-100 (9005-00-9), Talloweth-50, Talloweth-80 (61791-28-4), Trideceth-50 (24938-91-8),
the carboxylic acid polyglycol esters used are fatty acid polyglycol esters, preferably those with carboxylic esters of

8 to 20 carbon atoms, especially PEG-75 oleate, PEG-200 oleate, PEG-300 monooleate, PEG-400 oleate, PEG-150 laurate, PEG-400 laurate, PEG-75 stearate, PEG-100 stearate, PEG-600 stearate, PEG-150 distearate,
the ethoxylated sorbitan fatty acid esters used are PEG-40 sorbitan oleate, PEG-80 sorbitan laurate, the ethoxylated castor oil or hydrogenated variants used are PEG 75 castor oil or PEG200 castor oil or PEG-80 hydrogenated castor oil, PEG-100 hydrogenated castor oil, PEG-200 hydrogenated castor oil,
the ethoxylated fatty amines used are PEG-100 tallowalkyl-amine (61791-44-4), PEG-40 stearylamine, and
the ethoxylated glyceryl fatty acid carboxylates used are PEG-40 glyceryl laurate, PEG-200 glyceryl stearate, PEG-200 glyceryl tallowate and PEG-200 hydrogenated glyceryl palmate.

**8.** Aqueous emulsions according to any of Claims 1 to 7, **characterized in that** the carbamato-functionalized organopolysiloxanes (A) are prepared by reacting amino-functionalized organopolysiloxanes (A') containing an average per molecule of at least one group Y' of the formula

$$-R^4-[NH-R^5-]_nNH_2$$

where $R^4$, $R^5$ and n are as defined in Claim 1 with cyclic carbonates of the formula

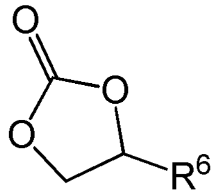

where
$R^6$ is as defined in Claim 1 or 3.

**9.** Aqueous emulsions according to Claim 8, **characterized in that** the amino-functionalized organopolysiloxanes (A') used are amino-functionalized polydiorganosiloxanes (A') of the formula

$$[R^1{}_2R^2SiO_{1/2}]_2[R^2(Y')SiO_{2/2}]_k[R^1{}_2SiO_{2/2}]_m \qquad (I')$$

where

$R^1$, $R^2$, m and k are as defined in Claim 2 and
Y' is as defined in Claim 8.

**10.** Aqueous emulsions according to Claim 8 or 9, **characterized in that** the amino-functionalized organopolysiloxanes (A') used are those having an amine value of at most 0.4 mmol/g, especially at most 0.2 mmol/g.

**11.** Cosmetic compositions comprising,
in a cosmetically acceptable medium, preferably water,
at least one hair conditioner and
at least one aqueous emulsion according to any of Claims 1 to 7 or produced according to any of Claims 8 to 10.

**12.** Cosmetic compositions according to Claim 11, **characterized in that** the hair conditioners used are those selected from the group of
cationic polymers,
cationic surfactants,
non-polymeric quaternary ammonium compounds, organopolysiloxanes and organopolysiloxane copolymers other than the carbamato-functionalized polydiorganosiloxanes (A) present in the aqueous emulsions,
fatty acid esters and fatty acid alcohols,
natural or synthetic oils and waxes and
panthenol, lipids, proteins and hydrolyzed proteins, and mixtures thereof.

**13.** Cosmetic compositions according to Claim 11 or 12, **characterized in that** they comprise further standard cosmetic additives selected from the group of surfactants, such as anionic, nonionic and amphoteric surfactants,

thickeners, gelating agents,
film formers,
humectants,
UV filters,
pearlizing agents,
vitamins,
antioxidants,
caffeine,
active antidandruff ingredients,
preservatives
and mixtures thereof.

**14.** Process for producing the cosmetic composition by mixing at least one aqueous emulsion of carbamato-functionalized organopolysiloxanes according to any of Claims 1 to 7 or produced according to any of Claims 8 to 10 with at least one conditioner
and optionally further standard cosmetic additives in a cosmetically acceptable medium, preferably water.

**15.** Process for treatment of keratinic fibers, preferably hair, with cosmetic compositions according to any of Claims 11 to 13 or produced according to Claim 14.

## Revendications

**1.** Émulsions aqueuses contenant

(A) des organopolysiloxanes fonctionnalisés par carbamato, qui contiennent en moyenne, par molécule, au moins un groupe fonctionnel Y de type carbamato de formule

$$-R^4-[NX-R^5-]_nNX-H,$$

X étant identique ou différent et signifiant un atome d'hydrogène ou un radical Z de formule

$$-CO-O-CHR^6-CH_2-OH$$

ou

$$-CO-O-CH_2-CHR^6-OH ,$$

en moyenne, par molécule, au moins un radical X étant un radical Z,
$R^4$ étant identique ou différent et signifiant un radical hydrocarboné lié par Si-C, divalent, comportant 1 à 18 atome(s) de C,
$R^5$ étant identique ou différent et signifiant un radical hydrocarboné divalent comportant 1 à 6 atome(s) de C,
$R^6$ étant identique ou différent et signifiant un atome d'hydrogène ou un radical hydrocarboné monovalent comportant 1 à 36 atome(s) de C et
n étant égal à 1, 2, 3 ou 4, de préférence égal à 1,
étant entendu que dans les organopolysiloxanes (A) fonctionnalisés par carbamato, moins de 50 % en moles des radicaux X N-liés dans les groupes Y ne sont pas un atome d'hydrogène, mais possèdent la signification ci-dessus du radical Z,

(B) des émulsifiants non ioniques contenant du poly(oxyde d'éthylène), qui contiennent plus de 40 motifs de type oxyde d'éthylène de formule $-CH_2-CH_2-O$ et présentent une valeur HLB supérieure ou égale à 15, choisis dans le groupe
des alkylpolyglycoléthers,
des esters polyglycoliques d'acides carboxyliques,
des esters d'acides gras de sorbitane éthoxylés, de l'huile de ricin éthoxylée ou des variantes hydrogénées,
des amines grasses éthoxylées,
des carboxylates d'acides gras de glycéryle éthoxylés,

des copolymères à bloc de motifs de type oxyde d'éthylène et oxyde de propylène, qui sont désignés comme poloxamères, et
des copolymères de motifs de type oxyde d'éthylène et oxyde de propylène, qui sont pontés par l'intermédiaire d'un noyau éthylènediamine et qui sont désignés comme poloxamines,
et
(C) de l'eau,
les émulsions présentant des grosseurs de particule inférieures ou égales à 100 nm (D50), mesurées selon la description.

2. Émulsions aqueuses selon la revendication 1, **caractérisées en ce que** des polydiorganosiloxanes fonctionnalisés par carbamato de formule

$$[R^1{}_2R^2SiO_{1/2}]_2[R^2(Y)SiO_{2/2}]_k[R^1{}_2SiO_{2/2}]_m \qquad (I),$$

$R^1$ étant identique ou différent et signifiant un radical hydrocarboné lié par Si-C, monovalent, comportant 1 à 18 atome(s) de C,
$R^2$ étant identique ou différent et étant égal au radical $R^1$ ou signifiant un groupe hydroxy -OH ou un groupe alcoxy de formule -O-$R^3$, $R^3$ étant un radical alkyle éventuellement substitué comportant 1 à 8 atome(s) de C,
Y possédant la signification indiquée pour celui-ci dans la revendication 1,
en moyenne, par molécule, au moins un groupe Y contenant un radical Z de formule

$$-CO-O-CHR^6-CH_2-OH$$

ou

$$-CO-O-CH_2-CHR^6-OH$$

$R^6$ possédant la signification indiquée pour celui-ci dans la revendication 1,
m étant un nombre entier et étant au moins 40 et au plus 1000
k étant un nombre entier et étant au moins 1 et au plus 15,
le rapport de m à k étant d'au moins 65 et d'au plus 1000,
sont utilisés en tant qu'organopolysiloxanes (A) fonctionnalisés par carbamato.

3. Émulsions aqueuses selon la revendication 1 ou 2, **caractérisées en ce que** $R^6$ est un radical méthyle.

4. Émulsions aqueuses selon la revendication 1, 2 ou 3, **caractérisées en ce que** dans les organopolysiloxanes (A) fonctionnalisés par carbamato, au moins 5 % en moles des radicaux X N-liés dans les groupes Y ne sont pas un atome d'hydrogène, mais possèdent la signification du radical Z de formule

$$-C-C)-O-CHR^6-CH_2-OH$$

ou

$$-CO-O-CH_2-CHR^6-OH$$

$R^6$ possédant la signification indiquée pour celui-ci dans la revendication 1.

5. Émulsions aqueuses selon l'une quelconque des revendications 1 à 4, **caractérisées en ce que** les organopolysiloxanes (A) fonctionnalisés par carbamato présentent au plus 0,7 % en poids, de préférence au plus 0,32 % en poids, préférablement au plus 0,17 % en poids de groupes hydroxy.

6. Émulsions aqueuses selon l'une quelconque des revendications 1 à 5, **caractérisées en ce que** les organopolysiloxanes (A) fonctionnalisés par carbamato présentent un indice d'amine d'au plus 0,3 mmol/g, particulièrement préférablement d'au plus 0,10 mmol/g.

7. Émulsions aqueuses selon l'une quelconque des revendications 1 à 6, **caractérisées en ce qu'**en tant qu'émulsifiants non ioniques contenant du poly(oxyde d'éthylène), qui contiennent plus de 40 motifs oxyde d'éthylène de

formule $-CH_2-CH_2-O-$ et présentent une valeur HLB supérieure ou égale à 15, ceux choisis dans le groupe
des alkylpolyglycoléthers comportant des radicaux alkyle de 8 à 20 atomes de carbone, préférablement le steareth-100 (9005-00-9), le talloweth-50, le talloweth-80 (61791-28-4), le trideceth-50 (24938-91-8),
en tant qu'esters polyglycoliques d'acides carboxyliques, des esters polyglycoliques d'acides gras, préférablement ceux comportant des esters d'acide carboxylique de 8 à 20 atomes de C, en particulier l'oléate de PEG-75, l'oléate de PEG-200, le monooléate de PEG-300, l'oléate de PEG-400, le laurate de PEG-150, le laurate de PEG-400, le stéarate de PEG-75, le stéarate de PEG-100, le stéarate de PEG-600, le distéarate de PEG-150,
en tant qu'esters d'acides gras de sorbitane éthoxylés, l'oléate de sorbitane de PEG-40, le laurate de sorbitane de PEG-80,
en tant qu'huile de ricin éthoxylée ou des variantes hydrogénées, l'huile de ricin PEG 75 ou l'huile de ricin PEG200, respectivement l'huile de ricin hydrogénée PEG-80, l'huile de ricin hydrogénée PEG-100, l'huile de ricin hydrogénée PEG-200,
en tant qu'amines grasses éthoxylées, l'alkylamine de suif de PEG-100 (61791-44-4), la stéarylamine de PEG-40, et
en tant que carboxylates d'acides gras de glycéryle éthoxylés, le laurate de glycéryle de PEG-40, le stéarate de glycéryle de PEG-200, le tallowate de glycéryle de PEG-200 et le palmitate de glycéryle hydrogéné de PEG-200, sont utilisés.

**8.** Émulsions aqueuses selon l'une quelconque des revendications 1 à 7, **caractérisées en ce que** les organopolysiloxanes (A) fonctionnalisés par carbamato sont préparés par transformation d'organopolysiloxanes (A') fonctionnalisés par amino, qui contiennent en moyenne par molécule au moins un groupe Y' de formule

$$-R^4-[NH-R^5-]_nNH_2,$$

$R^4$, $R^5$ et n possédant la signification indiquée pour ceux-ci dans la revendication 1, avec des carbonates cycliques de formule

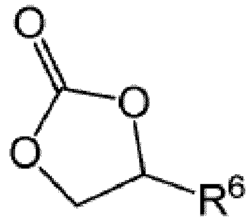

,

$R^6$ possédant la signification indiquée pour celui-ci dans la revendication 1 ou 3.

**9.** Émulsions aqueuses selon la revendication 8, **caractérisées en ce que** qu'en tant qu'organopolysiloxanes (A') fonctionnalisés par amino, des polydiorganosiloxanes (A') fonctionnalisés par amino de formule

$$[R^1{}_2R^2SiO_{1/2}]_2[R^2(Y')SiO_{2/2}]_k[R^1{}_2SiO_{2/2}]_m \qquad (I'),$$

$R^1$, $R^2$, m et k possédant la signification indiquée pour ceux-ci dans la revendication 2 et
Y' possédant la signification indiquée pour celui-ci dans la revendication 8, sont utilisés.

**10.** Émulsions aqueuses selon la revendication 8 ou 9, **caractérisées en ce qu'**en tant qu'organopolysiloxanes (A') fonctionnalisés par amino, ceux qui présentent un indice d'amine d'au plus 0,4 mmol/g, en particulier d'au plus 0,2 mmol/g, sont utilisés.

**11.** Compositions cosmétiques contenant, dans un milieu acceptable sur le plan cosmétique, de préférence de l'eau, au moins un agent de conditionnement capillaire et au moins une émulsion aqueuse selon l'une quelconque des revendications 1 à 7 ou préparée selon l'une quelconque des revendications 8 à 10.

**12.** Compositions cosmétiques selon la revendication 11, **caractérisées en ce qu'**en tant qu'agents de conditionnement capillaire, ceux choisis dans le groupe
des polymères cationiques,
des tensioactifs cationiques,
des composés d'ammonium quaternaires ne se trouvant pas sous forme de polymère,

des organopolysiloxanes et des copolymères d'organopolysiloxane, qui sont différents des polydiorganosiloxanes (A) fonctionnalisés par carbamato se trouvant dans les émulsions aqueuses, des esters d'acides gras et des alcools d'acides gras,
des huiles et des cires naturelles ou synthétiques et du panthénol, des lipides, des protéines et des protéines hydrolysées, ainsi que leurs mélanges, sont utilisés.

**13.** Compositions cosmétiques selon la revendication 11 ou 12, **caractérisées en ce qu'**elles contiennent d'autres additifs cosmétiques habituels, choisis dans le groupe
des tensioactifs, tels que les tensioactifs anioniques, non ioniques et amphotères,
des épaississants, des gélifiants,
des agents filmogènes,
des agents d'hydratation,
de filtre UV,
des agents nacrants,
des vitamines,
des antioxydants,
de la caféine,
des agents actifs antipelliculaires,
des conservateurs
et leurs mélanges.

**14.** Procédé pour la préparation de la composition cosmétique par le mélange d'au moins une émulsion aqueuse d'organopolysiloxanes fonctionnalisés par carbamato selon l'une quelconque des revendications 1 à 7 ou préparée selon l'une quelconque des revendications 8 à 10 avec au moins un agent de conditionnement et éventuellement d'autres additifs cosmétiques habituels dans un milieu acceptable sur le plan cosmétique, de préférence dans de l'eau.

**15.** Procédé pour le traitement de fibres kératiniques, préférablement de cheveux, par des compositions cosmétiques selon l'une quelconque des revendications 11 à 13 ou préparées selon la revendication 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 639369 A1
- EP 1972330 A
- WO 2009150213 A
- US 4620878 A
- US 6153569 A
- US 20120270985 A
- WO 2009150213 A1
- JP 2047371 A
- US 3642858 A
- DE 102010020192


### In der Beschreibung aufgeführte Nicht-Patentliteratur


- **HANS-DIETER DÖRFLER.** Grenzflächen- und Kolloidchemie. VCH, 1994, 198
- Ullmann's Encyclopedia of Industrial Chemistry, CD-ROM-Ausgabe. Wiley-VCH Verlag, 2003
- **K. KRUMMEL ; STEPHANE CHIRON ; J. JACHOWICZ.** The Chemistry and Manufacture of Cosmetics. 2000, vol. II, 359-396
- The Personal Care Products Council (formerly. International Cosmetic Ingredient Dictionary and Handbook. The Cosmetic, Toiletry, and Fragrance Association (CTFA), 2010
- **K. SCHRADER ; A. DOMSCH.** Cosmetology - Theory and Practice. Verlag für chemische Industrie, 2005, vol. II, II-8-II-2
- **G. ENGELHARDT ; H. JANCKE.** Über die 1H-, 13C- und 29Si-NMR chemischen Verschiebungen einiger linearer, verzweigter und cyclischer Methyl-Siloxan-Verbindungen. *J. Organometal. Chem.,* 1971, vol. 28, 293-300
- NMR spectroscopy of organosilicon compounds. **ELIZABETH A. WILLIAMS.** The Chemistry of Organic Silicon Compounds. John Wiley and Sons Ltd, 1989, 511-533
- **Y. K. KAMATH ; HANS-DIETRICH WEIGMANN.** *J. Soc. Cosmet. Chem.,* 1986, vol. 37, 111-124